# EUROPEAN PATENT APPLICATION

(11) **EP 1 382 688 A1**
(43) Date of publication of application: **21.01.2004**
(21) Application number: 02722727.1
(22) Date of filing: 24.04.2002
(51) Int. Cl.: C12Q 1/26, A61K 39/395, A61K 35/32, A61P 19/00, A61P 19/08, A61P 19/10, C12N 15/53

(54) **SCREENING METHOD**

(30) Priority: 25.04.2001 JP 2001128078
(71) Applicant: Takeda Chemical Industries, Ltd., Osaka-shi, Osaka 541-0045 (JP)
(72) Inventor: TANI, Akiyoshi, Osaka-shi, Osaka 546-0033 (JP); HONDA, Susumu, Nishinomiya-shi, Hyogo 662-0945 (JP); YASUMA, Tsuneo, Ibaraki-shi, Osaka 567-0011 (JP)
(74) Representative: Rickard, Timothy Mark Adrian
(86) International application number: PCT/JP2002/004055
(87) International publication number: WO 2002/088384

(57) **Abstract**

A method for screening a compound acting on a bone or its salt characterized by using NRH: quinone oxidoreductase (NQO2), its peptide fragment or a salt thereof; agents acting on a bone comprising compounds obtained by the above method; a method of preventing/treating human or mammalian bone diseases involving the inhibition or promotion of the expression or activity of NQO2; and diagnostics for bone diseases containing a polynucleotide encoding NQO2 or its peptide fragment or an antibody having an affinity specifically for NQO2, its peptide fragment or a salt thereof.

SEQUENCE LISTING

<110>  Takeda Chemical Industries, Ltd.

<120>  Screening Method

<130>  663006

<150>  JP 2001-128078
<151>  2001-04-25

<160>  8

<170>  PatentIn version 3.1

<210>  1
<211>  976
<212>  DNA
<213>  Homo sapiens

<220>
<221>  CDS
<222>  (176)..(868)
<223>

<400>  1
cccggaacct ggcgcaactc ctagagcggt ccttggggag acgcgggtcc cagtcctgcg      60

gctcctactg gggagtgcgc tggtcggaag attgctggac tcgctgaaga gagactacgc      120

aggaaagccc cagccaccca tcaaatcaga gagaaggaat ccaccttctt acgct atg 178
Met
1

gca ggt aag aaa gta ctc att gtc tat gca cac cag gaa ccc aag tct 226
Ala Gly Lys Lys Val Leu Ile Val Tyr Ala His Gln Glu Pro Lys Ser
            5                   10                  15

ttc aac gga tcc ttg aag aat gtg gct gta gat gaa ctg agc agg cag 274
Phe Asn Gly Ser Leu Lys Asn Val Ala Val Asp Glu Leu Ser Arg Gln
        20                  25                  30

ggc tgc acc gtc aca gtg tct gat ttg tat gcc atg aac ttt gag ccg 322
Gly Cys Thr Val Thr Val Ser Asp Leu Tyr Ala Met Asn Phe Glu Pro
    35                  40                  45

agg gcc aca gac aaa gat atc act ggt act ctt tct aat cct gag gtt 370
Arg Ala Thr Asp Lys Asp Ile Thr Gly Thr Leu Ser Asn Pro Glu Val
50                  55                  60                  65

ttc aat tat gga gtg gaa acc cac gaa gcc tac aag caa agg tct ctg 418
Phe Asn Tyr Gly Val Glu Thr His Glu Ala Tyr Lys Gln Arg Ser Leu
                70                  75                  80

gct agc gac atc act gat gag cag aaa aag gtt cgg gag gct gac cta 466
Ala Ser Asp Ile Thr Asp Glu Gln Lys Lys Val Arg Glu Ala Asp Leu
            85                  90                  95

gtg ata ttt cag ttc ccg ctg tac tgg ttc agc gtg ccg gcc atc ctg 514
Val Ile Phe Gln Phe Pro Leu Tyr Trp Phe Ser Val Pro Ala Ile Leu
        100                 105                 110

aag ggc tgg atg gat agg gtg ctg tgc cag ggc ttt gcc ttt gac atc 562
Lys Gly Trp Met Asp Arg Val Leu Cys Gln Gly Phe Ala Phe Asp Ile
    115                 120                 125

cca gga ttc tac gat tcc ggt ttg ctc cag ggt aaa cta gcg ctc ctt 610
Pro Gly Phe Tyr Asp Ser Gly Leu Leu Gln Gly Lys Leu Ala Leu Leu
130                 135                 140                 145

tcc gta acc acg gga ggc acg gcc gag atg tac acg aag aca gga gtc 658
Ser Val Thr Thr Gly Gly Thr Ala Glu Met Tyr Thr Lys Thr Gly Val
                150                 155                 160

aat gga gat tct cga tac ttc ctg tgg cca ctc cag cat ggc aca tta 706
Asn Gly Asp Ser Arg Tyr Phe Leu Trp Pro Leu Gln His Gly Thr Leu
            165                 170                 175

cac ttc tgt gga ttt aaa gtc ctt gcc cct cag atc agc ttt gct cct 754
His Phe Cys Gly Phe Lys Val Leu Ala Pro Gln Ile Ser Phe Ala Pro
        180                 185                 190

gaa att gca tcc gaa gaa gaa aga aag ggg atg gtg gct gcg tgg tcc 802
Glu Ile Ala Ser Glu Glu Glu Arg Lys Gly Met Val Ala Ala Trp Ser
    195                 200                 205

cag agg ctg cag acc atc tgg aag gaa gag ccc atc ccc tgc aca gcc 850

Gln Arg Leu Gln Thr Ile Trp Lys Glu Glu Pro Ile Pro Cys Thr Ala
210                 215                 220                 225

cac tgg cac ttc ggg caa taactctgtg gcacgtgggc atcacgtaag          898
His Trp His Phe Gly Gln
                230

cagcacacta ggaggcccag gcgcaggcaa agagaagatg gtgctgtcat gaaataaaat    958

tacaacatag ctacctgg                                                  976

<210> 2
<211> 231
<212> PRT
<213> Homo sapiens

<400> 2
Met Ala Gly Lys Lys Val Leu Ile Val Tyr Ala His Gln Glu Pro Lys
1               5                   10                  15

Ser Phe Asn Gly Ser Leu Lys Asn Val Ala Val Asp Glu Leu Ser Arg
            20                  25                  30

Gln Gly Cys Thr Val Thr Val Ser Asp Leu Tyr Ala Met Asn Phe Glu
        35                  40                  45

Pro Arg Ala Thr Asp Lys Asp Ile Thr Gly Thr Leu Ser Asn Pro Glu
    50                  55                  60

Val Phe Asn Tyr Gly Val Glu Thr His Glu Ala Tyr Lys Gln Arg Ser
65                  70                  75                  80

Leu Ala Ser Asp Ile Thr Asp Glu Gln Lys Lys Val Arg Glu Ala Asp
                85                  90                  95

Leu Val Ile Phe Gln Phe Pro Leu Tyr Trp Phe Ser Val Pro Ala Ile
            100                 105                 110

Leu Lys Gly Trp Met Asp Arg Val Leu Cys Gln Gly Phe Ala Phe Asp
        115                 120                 125

Ile Pro Gly Phe Tyr Asp Ser Gly Leu Leu Gln Gly Lys Leu Ala Leu
    130                 135                 140

Leu Ser Val Thr Thr Gly Gly Thr Ala Glu Met Tyr Thr Lys Thr Gly
145                 150                 155                 160

Val Asn Gly Asp Ser Arg Tyr Phe Leu Trp Pro Leu Gln His Gly Thr
                165                 170                 175

Leu His Phe Cys Gly Phe Lys Val Leu Ala Pro Gln Ile Ser Phe Ala
            180                 185                 190

Pro Glu Ile Ala Ser Glu Glu Glu Arg Lys Gly Met Val Ala Ala Trp
        195                 200                 205

Ser Gln Arg Leu Gln Thr Ile Trp Lys Glu Glu Pro Ile Pro Cys Thr

210                    215                    220

Ala His Trp His Phe Gly Gln
225                    230

<210>  3
<211>  999
<212>  DNA
<213>  Mus musculus

<220>
<221>  CDS
<222>  (152)..(844)
<223>

<400>  3
ccttctgaat catgcctgcc tggatactgc catgctccca ccttgatata atggactgaa      60

cctctgaacc tagtgctaca ataaccaagg agagtaacag taccgaaact tttggctgtt     120

ataccaaatc agagaatcta tctcctccaa c atg gca ggt aag aaa gtg ctc        172
                                   Met Ala Gly Lys Lys Val Leu
                                   1               5

atc gtc tat gca cac caa gaa ccc aag tcc ttc aat ggg tcc ctg aag       220
Ile Val Tyr Ala His Gln Glu Pro Lys Ser Phe Asn Gly Ser Leu Lys
        10                  15                  20

aaa gtg gct gtt gaa gaa ctg agc aag cag gga tgc aca gtc act gtg 268
Lys Val Ala Val Glu Glu Leu Ser Lys Gln Gly Cys Thr Val Thr Val
    25                  30                  35

tct gat tta tat agc atg aac ttt gag cca agg gcc aca aga aat gat 316
Ser Asp Leu Tyr Ser Met Asn Phe Glu Pro Arg Ala Thr Arg Asn Asp
40                  45                  50                  55

atc act ggt gcc ccc tct aat cct gac gtc ttc agt tat ggg ata gaa 364
Ile Thr Gly Ala Pro Ser Asn Pro Asp Val Phe Ser Tyr Gly Ile Glu
                60                  65                  70

acc cat gaa gcc tac aag aag aaa gct ctg acc agt gat ata ttt gaa 412
Thr His Glu Ala Tyr Lys Lys Lys Ala Leu Thr Ser Asp Ile Phe Glu
            75                  80                  85

gaa cag aga aag gtg caa gaa gct gat ctt gtg ata ttt cag ttt cca 460
Glu Gln Arg Lys Val Gln Glu Ala Asp Leu Val Ile Phe Gln Phe Pro
        90                  95                  100

cta tac tgg ttc agc gtt cca gca atc cta aaa ggt tgg atg gat agg 508
Leu Tyr Trp Phe Ser Val Pro Ala Ile Leu Lys Gly Trp Met Asp Arg
    105                 110                 115

gtg ctg tgc cga ggg ttt gcc ttt gat atc cca ggc ttt tat gac tct 556
Val Leu Cys Arg Gly Phe Ala Phe Asp Ile Pro Gly Phe Tyr Asp Ser
120                 125                 130                 135

ggt ttt ctc aag ggt aaa tta gct ctc ctt tcc tta acc acg gga ggt 604

Gly Phe Leu Lys Gly Lys Leu Ala Leu Leu Ser Leu Thr Thr Gly Gly
                140                 145                 150

aca gcg gag atg tac aca aaa gat ggg gtc agt gga gat ttc cgg tac 652
Thr Ala Glu Met Tyr Thr Lys Asp Gly Val Ser Gly Asp Phe Arg Tyr
            155                 160                 165

ttc ctg tgg cca ctt cag cat ggt aca ctg cac ttc tgt gga ttt aaa 700
Phe Leu Trp Pro Leu Gln His Gly Thr Leu His Phe Cys Gly Phe Lys
        170                 175                 180

gtc ctt gcc ccc cag atc agt ttt ggt ctt gat gtt tca tca gaa gaa 748
Val Leu Ala Pro Gln Ile Ser Phe Gly Leu Asp Val Ser Ser Glu Glu
    185                 190                 195

gaa agg aaa gtg atg ctg gca tca tgg gcc cag cgg ctg aag agc atc 796
Glu Arg Lys Val Met Leu Ala Ser Trp Ala Gln Arg Leu Lys Ser Ile
200                 205                 210                 215

tgg aag gaa gaa ccc atc cac tgc aca ccc cct tgg tac ttc caa gag 844
Trp Lys Glu Glu Pro Ile His Cys Thr Pro Pro Trp Tyr Phe Gln Glu
                220                 225                 230

taacattttt gtgctctgag tacagctgac aagcaacaca gtgagagacc tacagcatgt 904

gcaaagagaa ggtggtgttg tatcctgaga tgtatttaac agtgcccacc aatgagtgtc 964

ttcagtttaa tacaactagt tcagatattt caaaa 999

<210> 4

<211> 231

<212> PRT

<213> Mus musculus

<400> 4

Met Ala Gly Lys Lys Val Leu Ile Val Tyr Ala His Gln Glu Pro Lys
1               5                   10                  15

Ser Phe Asn Gly Ser Leu Lys Lys Val Ala Val Glu Glu Leu Ser Lys
            20                  25                  30

Gln Gly Cys Thr Val Thr Val Ser Asp Leu Tyr Ser Met Asn Phe Glu
        35                  40                  45

Pro Arg Ala Thr Arg Asn Asp Ile Thr Gly Ala Pro Ser Asn Pro Asp
    50                  55                  60

Val Phe Ser Tyr Gly Ile Glu Thr His Glu Ala Tyr Lys Lys Lys Ala
65                  70                  75                  80

Leu Thr Ser Asp Ile Phe Glu Glu Gln Arg Lys Val Gln Glu Ala Asp
                85                  90                  95

Leu Val Ile Phe Gln Phe Pro Leu Tyr Trp Phe Ser Val Pro Ala Ile
            100                 105                 110

Leu Lys Gly Trp Met Asp Arg Val Leu Cys Arg Gly Phe Ala Phe Asp

        115                 120                 125

Ile Pro Gly Phe Tyr Asp Ser Gly Phe Leu Lys Gly Lys Leu Ala Leu

    130                 135                 140

Leu Ser Leu Thr Thr Gly Gly Thr Ala Glu Met Tyr Thr Lys Asp Gly

145                 150                 155                 160

Val Ser Gly Asp Phe Arg Tyr Phe Leu Trp Pro Leu Gln His Gly Thr

                165                 170                 175

Leu His Phe Cys Gly Phe Lys Val Leu Ala Pro Gln Ile Ser Phe Gly

            180                 185                 190

Leu Asp Val Ser Ser Glu Glu Glu Arg Lys Val Met Leu Ala Ser Trp

        195                 200                 205

Ala Gln Arg Leu Lys Ser Ile Trp Lys Glu Glu Pro Ile His Cys Thr

    210                 215                 220

Pro Pro Trp Tyr Phe Gln Glu

225                 230

<210> 5

<211> 29

<212> DNA

<213> Artificial

<220>

<221> misc_feature

<223> Oligonucleotide designed to act as primer for amplifying mNQO2
      cDNA.

<400> 5
ccggatccat ggcaggtaag aaagtgctc                                    29

<210> 6
<211> 28
<212> DNA
<213> Artificial

<220>

<221> misc_feature

<223> Oligonucleotide designed to act as primer for amplifying mNQO2
      cDNA.

<400> 6
ccgtcgactt actcttggaa gtaccaag                                     28

<210> 7
<211> 20
<212> DNA
<213> Artificial

<220>

<221>  misc_feature
<223>  Antisense oligonucleotide for mNQO2 mRNA.

<400>  7
ctgccatgtt ggaggagata                                                    20

<210>  8
<211>  20
<212>  DNA
<213>  Artificial

<220>
<221>  misc_feature
<223>  Sense oligonucleotide for mNQO2 mRNA.

<400>  8
tatctcctcc aacatggcag                                                    20

## Description

### Technical Field

The present invention relates to a screening method for a drug for treating bone diseases, which comprises using NRH: quinone oxidoreductase (NQO2), and the like.

### Background Art

Bone diseases include non-metabolic bone diseases such as bone fractures, bone/spinal deformation, osteosarcoma, myeloma, bone dysplasia, scoliosis, etc.; and metabolic bone diseases such as osteoporosis, osteomalacia, rickets, fibrous osteitis, renal bone dystrophy, Paget's disease of bone, etc. Recently, the metabolic bone diseases have become more problematic. For example, osteoporosis, one of metabolic bone diseases, is a systemic disease characterized by an increase in bone fragility and fracturability caused by a reduction in bone mass and a change in fine bone tissue structures, wherein its major clinical symptoms include spinal kyphosis, and fractures of dorsolumbar bones, vertebral centra, femoral necks, lower end of radius, ribs, upper end of humerus, and others. In bone tissues, bone formation and bone destruction caused by bone resorption are repeated with maintaining their balance, wherein osteoblasts including osteoblast precursors play key roles in osteogenesis and osteoclasts do so in bone resorption. When the balance is lost between the osteogenesis and the bone destruction caused by the bone resorption, a reduction in bone mass is resulted. Heretofore, as a drug for preventing or treating osteoporosis, bone resorption suppressors such as estrogen agents, calcitonin and bisphosphonates have been mainly used.

Further, articular diseases are mainly caused by the degeneration of articular cartilage (for example, rheumatoid arthritis, osteoarthritis, etc.). The articular cartilage is a tissue composed of collagen and proteoglycan. Many causes can lead to acceleration of release of the proteoglycan from the articular cartilage tissue and lead to the start of a reduction in the proteoglycan synthetic capability of the tissue. At the same time, the release and activation of matrix metalloprotease such as collagenase-3 are promoted so that collagen can be decomposed in the cartilage tissue. A series of these reactions promote the consolidation and destruction of the cartilage tissue, and then the lesion develops into synovial membrane hyperplasia, destruction of lower end cartilage, hypertrophy of articular verge cartilage, or bone neogenesis. In a severe case, the lesion can lead to dysfunction via articular deformation. The articular diseases occur in knee joints with a highest frequency but also occur in elbow, hip, foot, or finger joints. Of the articular diseases, the most frequently suffered disease is osteoarthritis, and one cause of such a disease may be aging. Therefore, the future aging society is expected to have an increased number of such patients. In the treatment, analgesic-anti-inflammatory agents or hyaluronic acid preparations are used for the purpose of removing the pain accompanied by the cartilage degeneration and the consolidation and destruction of the lower end cartilage. However, any of these pharmaceuticals is merely used for symptomatic treatment and not sufficiently effective. It is considered to be effective for preventing or treating cartilage diseases to promote chondrogenesis, to inhibit destruction of cartilage and to promote induction of differentiation of chondrocytes including precursor chondrocytes.

In clinical prevention or treatment of bone diseases or articular diseases (e.g. cartilage diseases), there has still been a demand for a pharmaceutical that can exhibit an excellent effect. In particular, there has been a demand for a pharmaceutical that is excellent in prevention or treatment of articular diseases whose representative examples include cartilage diseases.

### Objects of the Invention

It is an object of the present invention to provide a screening means for identifying biological substances involved in control of formation of bones (or cartilages) or differentiation of osteoblasts (or chondrocytes) and performing a screening for substances that can regulate the physiological activity of the biological substances, thereby providing pharmaceuticals that can have an excellent prophylactic or therapeutic effect on bone diseases (hereinafter, diseases of bones or joints are generically called "bone diseases").

### Summary of the Invention

The present inventors have studied intensively to solve the above problem and unexpectedly found that chromone derivatives showing a bone differentiation-inducing activity surprisingly inhibit the activity of NRH: quinone oxidoreductase (NQO2). Based on these findings, the present inventors have also found that NQO2 is useful for efficiently performing a screening for substances that have a prophylactic or therapeutic effect on bone diseases.

Thus, the inventors have further studied based on these findings and have completed the present invention.

Thus, the present invention provides:
(1) A method for screening a compound acting on a bone, or a salt thereof, which comprises using NRH: quinone oxidoreductase (NQO2), a peptide fragment thereof, or a salt thereof;
(2) The screening method according to the above (1), wherein the action on a bone is osteogenesis-promoting action, osteoblast differentiation-inducing action, osteoblast differentiation induction-promoting action, chondrogenesis-promoting action, chondrocyte differentiation-inducing action, chondrocyte differentiation induction-promoting action, or BMP action-enhancing action;
(3) The screening method according to the above (1), wherein the action on a bone is osteogenesis-inhibiting action, osteoblast differentiation-inhibiting action, osteoblast differentiation induction-inhibiting action, chondrogenesis-inhibiting action, chondrocyte differentiation-inhibiting action, chondrocyte differentiation induction-inhibiting action, or BMP action-suppressing action;
(4) The screening method according to the above (1), wherein a comparison is made between (i) the action on a bone in case where NQO2, a peptide fragment thereof, or a salt thereof is brought into contact with a substrate and (ii) that in case where NQO2, a peptide fragment thereof, or a salt thereof is brought into contact with the substrate and a test compound;
(5) A kit for screening a compound or a salt thereof acting on a bone, which comprises NRH: quinone oxidoreductase (NQO2), a peptide fragment thereof, or a salt thereof;
(6) A compound acting on a bone or a salt thereof obtainable by using the screening method according to the above (1) or the screening kit according to the above (5);
(7) A bone-acting agent, which comprises a compound or a salt thereof obtainable by using the screening method according to the above (1) or the screening kit according to the above (5);
(8) An agent for preventing and/or treating a bone disease, which comprises a compound having inhibitory action on the expression or activity of NRH: quinone oxidoreductase (NQO2), or a salt thereof;
(9) The agent according to the above (8), which is an osteogenesis-promoting agent, an osteoblast differentiation-inducing agent, an osteoblast differentiation induction-promoting agent, a chondrogenesis-promoting agent, a chondrocyte differentiation-inducing agent, a chondrocyte differentiation induction-promoting agent, or a BMP action-enhancing agent;
(10) The agent according to the above (8), which is an agent for preventing and/or treating bone fracture, bone refracture, bone/spinal deformation, osteosarcoma, myeloma, bone dysplasia, scoliosis, bone defect, osteoporosis, osteomalacia, rickets, fibrous osteitis, renal bone dystrophy, Paget's disease of bone, rigid myelitis, osteoarthritis, or rheumatoid arthritis;
(11) An agent for preventing and/or treating a bone disease, which comprises a compound having promoting action on the expression or activity of NRH: quinone oxidoreductase (NQO2), or a salt thereof;
(12) An agent for preventing and/or treating a bone disease, which comprises a polynucleotide having a nucleotide sequence complementary to a polynucleotide encoding NRH: quinone oxidoreductase (NQO2), or a fragment thereof;
(13) An agent for preventing and/or treating a bone disease, which comprises an antibody against NRH: quinone oxidoreductase (NQO2), a peptide fragment thereof, or a salt thereof;
(14) A diagnostic agent for a bone disease, which comprises an antibody against NRH: quinone oxidoreductase (NQO2), a peptide fragment thereof, or a salt thereof;
(15) A method for preventing and/or treating a mammalian bone disease, which comprises inhibiting or promoting the expression or activity of NRH: quinone oxidoreductase (NQO2) in a mammalian body to an extent effective for preventing or treating the bone disease;
(16) A method for preventing and/or treating a mammalian bone disease, which comprises administering a compound that inhibits or promotes the expression or activity of NRH: quinone oxidoreductase (NQO2), or a salt thereof, in an amount effective for preventing and/or treating the bone disease to a mammal; and
(17) Use of a compound that inhibits or promotes the expression or activity of NRH: quinone oxidoreductase (NQO2), or a salt thereof for the manufacture of an agent for preventing and/or treating a bone disease.

### Brief Description of Drawing

Fig. 1 shows the results of the measurement of an alkaline phosphatase activity in an osteoblast cell line MC3T3-E1 obtained in Example 2, into which antisense or sense DNA to NQO2 mRNA is introduced. The alkaline phosphatase activity is represented by a relative activity (%) by taking a control value (with no DNA) as 100.

### Detailed Description of the present invention

### [1] NQO2 Protein

NQO2 to be used for the screening method of the present invention (hereinafter referred to as the protein of the present invention in some cases) may be any enzyme in so far as it contains the amino acid sequence represented by SEQ ID NO: 2 or 4 or an amino acid sequence substantially identical therewith and has NRH: quinone oxidoreductase activity.

NQO2 may be a protein derived from any cells of a human being or other mammals (such as guinea pig, rat, mouse, rabbit, swine, sheep, bovine, monkey, etc.) such as splenocytes, nerve cells, glia cells, pancreas β cells, bone marrow cells, mesangial cells, Langerhans' cells, epidermic cells, epithelial cells, endothelial cells, fibroblasts, fibrocytes, myocytes, fat cells, immunocytes (e.g. macrophages, T cells, B cells, natural killer cells, mast cells, neutrophils, basophils, eosinophils, and monocytes), megakaryocytes, synovial cells, chondrocytes, bone cells, osteoblasts, osteoclasts, mammary gland cells, hepatocytes, interstitial cells, and corresponding precursor cells, stem cells and cancer cells, and blood cells, or derived from any tissue where such cells exist, for example, brain or any part thereof (e.g. olfactory bulb, amygdaloid nucleus, basal ganglia, hippocampus, thalamus, hypothalamus, subthalamic nucleus, cerebral cortex, medulla oblongata, cerebellum, occipital lobe of cerebrum, frontal lobe of cerebrum, temporal lobe, putamen, caudate nucleus, corpus callosum, and substantia nigra), spinal cord, hypophysis, stomach, pancreas, kidney, liver, gonad, thyroid, gallbladder, bone marrow, adrenal gland, skin, muscle, lung, gastrointestinal tract (e.g. large intestine and small intestine), blood vessel, heart, thymus, spleen, submandibular gland, peripheral blood, peripheral blood cell, prostate, testis, orchis, ovary, placenta, uterus, bone, joint, and skeletal muscle, or may be any synthetic proteins

Examples of the amino acid sequence substantially identical with the amino acid sequence represented by SEQ ID NO: 2 or 4 include amino acid sequences having a homology of about 50% or more, preferably about 60% or more, more preferably about 70% or more, further more preferably about 80% or more, yet further more preferably about 90% or more, most preferably about 95% or more with the amino acid sequence represented by SEQ ID NO: 2 or 4.

For example, the protein including the amino acid sequence substantially identical with the sequence represented by SEQ ID NO: 2 or 4 is preferably a protein having an amino acid sequence substantially identical with the sequence represented by SEQ ID NO: 2 or 4 and having an activity substantially equal in quality to that of the amino acid sequence represented by SEQ ID NO: 2 or 4.

For example, the activity substantially equal in quality includes NRH: quinone oxidoreductase activity. The wording "substantially equal in quality" means that the nature of activities are the same. Therefore, the activities may differ from each other in quantitative factors such as a degree of an activity and a molecular weight of a protein, while the activities such as oxidoreductase activity or the like are the same (e.g. in the range of about 0.01 to 100 times, preferably about 0.5 to 20 times, more preferably about 0.5 to 2 times).

The measurement of the activity such as NRH: quinone oxidoreductase activity may be performed according to a per se known method.

Further, as NQO2 to be used for the screening method of the present invention, a protein having (1) an amino acid sequence in which one or more amino acid residues (preferably 1 to 30, more preferably 1 to 10, further more preferably a few (1 to 5) amino acid residues) are deleted from the amino acid sequence represented by SEQ ID NO: 2 or 4, (2) an amino acid sequence in which one or more amino acid residues (preferably 1 to 30, more preferably 1 to 10, further more preferably a few (1 to 5) amino acid residues) are added to the amino acid sequence represented by SEQ ID NO: 2 or 4, (3) an amino acid sequence in which one or more amino acid residues (preferably 1 to 30, more preferably 1 to 10, further more preferably a few (1 to 5) amino acid residues) are replaced with any other residue(s) in the amino acid sequence represented by SEQ ID NO: 2 or 4, or (4) an amino acid sequence of any combination thereof, can also be used.

According to the conventional notation of peptides, the N terminal end (amino terminal end) of NQO2 is placed on the left side end, and the C terminal end (carboxyl terminal end) thereof on the right side end. In NQO2 to be used for the screening method of the present invention including NQO2 containing the amino acid sequence represented by SEQ ID NO: 2 or 4, usually, the C terminal end is a carboxyl group (-COOH) or carboxylate (-COO⁻), but the C terminal end may also be an amide (-CONH₂) or ester (-COOR) .

Examples of R in the ester include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂ alkyl group, for example, benzyl, phenethyl, etc., and an α-naphthyl-C₁₋₂ alkyl group, for example, α-naphthylmethyl, etc.; and further a pivaloyloxymethyl group, etc., which are ester generally used for oral administration.

When NQO2 has a carboxyl group (or carboxylate) at a position other than the C terminal end, such a carboxyl group may be amidated or esterified and they are also included in NQO2. As such an ester, the ester as mentioned above with respect to the C terminal end can be used.

Further, NQO2 also include modifications of the above proteins such as those in which the amino group of the N terminal methionine residue is protected by a protective group (e.g. a C₁₋₆ acyl group such as a formyl group and a C₂₋₆ alkanoyl group such as acetyl); those having a pyroglutamic acid group converted from a glutamyl group that is formed by cleaving the N terminus in vivo; those in which a substituent of a side chain of an amino acid in a molecule (e.g. -OH, -SH, an amino group, an imidazole group, an indole group, and a guanidino group) is protected by an appropriate protective group (e.g. a C₁₋₆ acyl group such as a formyl group and a C₂₋₆ alkanoyl group such as acetyl); as well as so-called conjugated proteins such as glycoproteins having sugar chains bonded.

Specific examples of NQO2 to be used for the screening method of the present invention include human-derived NQO2 having the amino acid sequence represented by SEQ ID NO: 2 and mouse-derived NQO2 having the amino acid sequence represented by SEQ ID NO: 4.

### [2] Peptide Fragment

A peptide fragment of NQO2 (hereinafter abbreviated to partial peptide in some cases) may be any partial peptide of the above NQO2 including, for example, that having NRH: quinone oxidoreductase activity.

As for the number of the amino acids in such a partial peptide, a preferred peptide is that having an amino acid sequence of at least 20, preferably 50 or more, more preferably 100 or more of those in the amino acid sequence constituting the above NQO2.

The substantially identical amino acid sequence means an amino acid sequence having a homology of about 50% or more, preferably about 60% or more, more preferably about 70% or more, further more preferably about 80% or more, yet further more preferably about 90% or more, most preferably about 95% or more with any of the above amino acid sequences.

The wording "the activity substantially equal in quality" used herein has the same meaning as that stated above. The measurement of "the activity substantially equal in quality" can be performed in the same manner as that described above.

Further, the partial peptide may include peptides having an amino acid sequence in which 1 or more amino acid residues (preferably 1 to 10, more preferably a few (1 to 5) amino acid residues) are deleted from the above amino acid sequence; an amino acid sequence in which 1 or more amino acid residues (preferably 1 to 20, more preferably 1 to 10, further more preferably a few (1 to 5) amino acid residues) are added to the above amino acid sequence; or an amino acid sequence in which 1 or more amino acid residues (preferably 1 to 10, more preferably several, further more preferably 1 to 5 amino acid residues) are replaced with other residue(s) in the above amino acid sequence.

Further, in the partial peptide, usually, the C terminal end is a carboxyl group (-COOH) or carboxylate (-COO⁻), but the C terminal end may be an amide (-CONH₂) or ester (-COOR) as in the same manner as that described above with respect to the protein of the present invention.

Furthermore, similarly to the above NQO2, the partial peptide may also include modifications such as those in which the amino group of the N terminal methionine residue is protected by the protective group; those having a pyroglutamic acid group converted from Gln formed by cleaving the N terminus in vivo; those in which a substituent of a side chain of an amino acid in a molecule is protected by an appropriate protective group; or so-called conjugated proteins such as glycoproteins having sugar chains bonded.

Further, in the partial peptide, usually, the C terminal end is a carboxyl group (-COOH) or carboxylate (-, COO⁻), but the C terminal end may be an amide (-CONH₂) or ester (-COOR) as in the same manner as that described above with respect to the protein of the present invention.

The salt of NQO2 or the partial peptide thereof may be a physiologically acceptable salt with an acid or base, in particular, a physiologically acceptable acid addition salt is preferred. Examples of the salt used include a salt with an inorganic acid (e.g. hydrochloric acid, phosphoric acid, hydrobromic acid, and sulfuric acid); and a salt with an organic acid (e.g. acetic acid, formic acid, propionic acid, fumaric acid, maleic acid, succinic acid, tartaric acid, citric acid, malic acid, oxalic acid, benzoic acid, methanesulfonic acid, and benzenesulfonic acid), etc.

### [3] Synthesis of NQO2 or Its Peptide Fragment

NQO2 or a salt thereof may be produced from the above cells or tissues of a human being or other mammals by a per se known purification technique, or produced by culturing a transformant carrying DNA encoding NQO2 as described hereinafter, or produced by in vitro synthesis using the DNA and a cell-free translation system derived from a rabbit reticulocyte extract or a wheat germ extract. It may also be produced by a protein synthesis method as described hereinafter or an analogous method.

When NQO2 or a salt thereof is produced from tissues or cells of a human being or other mammals, the tissues or cells of a human being or other mammals are homogenized and then subjected to ultrasonication, treatment with a surfactant or the like to obtain a cellular extract. The desired material can be purified from the extract by using an appropriate combination of conventional techniques for separation and purification of proteins. Examples of such separation techniques include solubility difference-based methods such as salting out and solvent precipitation method; molecular weight difference-based methods such as dialysis, ultrafiltration, gel filtration, non-denaturing polyacrylamide electrophoresis (PAGE), and sodium dodecyl sulfate-polyacrylamide electrophoresis (SDS-PAGE); electrical charge-based methods such as ion exchange chromatography and hydroxyapatite chromatography; specific affinity-based methods such as affinity chromatography; hydrophobic difference-based methods such as reverse phase high performance liquid chromatography; isoelectric point difference-based methods such as isoelectric focusing; and the like.

Usually, a commercially available resin for protein synthesis may be used for the synthesis of NQO2, a peptide fragment thereof, a salt thereof, or an amide derivative thereof. Examples of such a resin include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethyl-methylphenylacetamidomethyl resin, polyacrylamide resin, 4-(2',4'-dimethoxyphenyl-hydroxymethyl)phenoxy resin, and 4-(2',4'-dimethoxyphenyl-Fmoc-aminoethyl)phenoxy resin. By using such a resin, amino acids wherein α-amino groups and side chain functional groups have been appropriately protected are condensed on the resin in order of the desired protein sequence according to a per se known condensation method. At the final stage of the reaction, a protein is detached from the resin and, at the same time, various protective groups are removed. Further, an intramolecular disulfide-forming reaction is performed in a highly diluted solution to obtained the desired protein or an amide derivative thereof.

In the above condensation of protected amino acids, various activating reagents available for protein synthesis may be used, and carbodiimides are particularly preferred. Examples of the carbodiimides to be used include DCC, N,N'-diisopropylcarbodiimide, N-ethyl-N'-(3-dimethylaminoprolyl)carbodiimide, etc. For activation with such a reagent, a protected amino acid together with a racemization inhibitor (e.g. HOBt and HOOBt) may be directly added to the resin, or a protected amino acid may be activated beforehand in the form of a symmetric acid anhydride, a HOBt ester or a HOOBt ester, and then added to the resin.

A solvent to be used in the activation of protected amino acids and in the condensation thereof on the resin may be appropriately selected from known solvents applicable in condensation reactions of proteins. Examples of the solvent include acid amides such as N,N-dimethylformamide, N,N-dimethylacetamide and N-methylpyrrolidone; halogenated hydrocarbons such as methylene chloride and chloroform; alcohols such as trifluoroethanol; sulfoxides such as dimethyl sulfoxide; ethers such as pyridine, dioxane, and tetrahydrofuran; nitriles such as acetonitrile and propionitrile; esters such as methyl acetate and ethyl acetate; and an appropriate mixture thereof. The reaction temperature may be selected from a range known to be useful for protein bond-forming reactions, usually from the range of about-20°C to about 50°C. The activated amino acid derivative is usually used in 1.5 to 4-fold excess. In case that the condensation is found to be insufficient as indicated by a ninhydrin reaction test, the reaction can be repeated without removing protective groups to perform sufficient condensation. In case that sufficient condensation cannot be performed even by the repetition of the reaction, acetic anhydride or acetylimidazole may be used to acetylate unreacted amino acids.

Examples of the protective group for protecting the amino group of a starting material include Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphinothioyl, Fmoc, etc.

The carboxyl group may be protected, for example, by alkyl esterification (e.g. esterification by a straight or branched chain, or cyclic alkyl such as methyl, ethyl, propyl, butyl, tert-butyl, cyclopentyl, cyclohexyl, cycloheptyl, cyclooctyl, and 2-adamantyl esters); aralkyl esterification (e.g. benzyl esterification, 4-nitrobenzyl esterification, 4-methoxybenzyl esterification, 4-chlorobenzyl esterification, and benzhydryl esterification); phenacyl esterification; formation of benzyloxycarbonylhydrazide; formation of tert-butoxycarbonylhydrazide; or formation of tritylhydrazide.

For example, the hydroxyl group of serine may be protected by esterification or etherification. Examples of the group suitable for the esterification include a lower alkanoyl group such as acetyl; an aroyl group such as benzoyl; and a carbonic acid-derived group such as benzyloxycarbonyl and ethoxycarbonyl. Examples of the group suitable for the etherification include a benzyl group, a tetrahydropyranyl group, and a tert-butyl group.

Examples of the protective group for the phenolic hydroxyl group of tyrosine include Bzl, Cl₂-Bzl, 2-nitrobenzyl, Br-Z, and tert-butyl.

Examples of the protective group for the imidazole group of histidine include Tos, 4-methoxy-2,3,6-trimethylbenzenesulfonyl, DNP, benzyloxymethyl, Bum, Boc, Trt, and Fmoc.

Examples of the starting material having the activated carboxyl group to be used include a corresponding acid anhydride, azide, and active ester (ester with an alcohol such as pentachlorophenol, 2,4,5-trichlorophenol, 2,4-dinitrophenol, cyanomethyl alcohol, p-nitrophenol, HONB, N-hydroxysuccinimide, N-hydroxyphthalimide, and HOBt). The starting material having the activated amino group includes, for example, a corresponding phosphoramide.

Examples of the method for removing the protective group include catalytic reduction in a hydrogen stream in the presence of a catalyst such as Pd-black and Pd-carbon; treatment with an acid such anhydrous hydrogen fluoride, methanesulfonic acid, trifluoromethanesulfonic acid, trifluoroacetic acid, or any mixture thereof; treatment with a base such as diisopropylethylamine, triethylamine, piperidine, and piperazine; and reduction with sodium in liquid ammonia. The deprotection by the above acid treatment is generally performed at a temperature of about -20°C to 40°C. In this acid treatment, it is advantageous to add a cation scavenger such as anisole, phenol, thioanisole, m-cresol, p-cresol, dimethyl sulfide, 1,4-butanedithiol, and 1,2-ethanedithiol. Further, 2,4-dinitrophenyl group used for protecting the imidazole group of histidine can be removed by treatment with thiophenol. The formyl group used for protecting the indole group of tryptophan can be removed by alkali treatment with a diluted sodium hydroxide solution, a diluted liquid ammonia, or the like, in addition to the deprotection by the above acid treatment in the presence of 1,2-ethanedithiol, 1,4-butanedithiol or the like.

A known group or a known means may be appropriately selected for the protection of functional groups which are not involved in the reaction, for the protective groups, and for the removal of the protective groups, as well as for the activation of functional groups which are involved in the reaction, and the like.

An alternative method for obtaining the amide derivative of the protein comprises, for example, protecting the α-carboxyl group of the C-terminal amino acid by amidation; extending a peptide (protein) chain to the desired chain length towards the amino group side; removing the protecting group from the peptide chain to prepare a protein wherein only the protecting group of the N-terminal α-amino group is removed, and a protein only the protecting group of the C-terminal carboxyl group is removed; and condensing both proteins in a mixed solvent as mentioned above. The condensation reaction can be performed by the same manner as described above. After purification of the protected protein obtained by the condensation, all the protective groups can be removed by the above method to obtain the desired protein in a crude form. This crude protein is purified by making full use of suitable known purification techniques, and then a main fraction is lyophilized to obtain an amide derivative of the desired protein.

The ester derivative of the protein can be obtained by, for example, condensing the α-carboxyl group of the C-terminal amino acid with a suitable alcohol to form an amino acid ester; and then treating it according to the same procedure as described above with respect to the amide derivative of the protein to obtain an ester derivative of the desired protein.

The peptide fragment of NQO2 or a salt thereof may be produced by a known peptide synthesis technique or by cleaving NQO2 with any suitable peptidase. The peptide synthesis may be either of a solid-phase synthesis and a liquid-phase synthesis. That is, the desired peptide can be produced by condensing a partial peptide or amino acids capable of constituting NQO2 with a residual part thereof and optionally removing protective group(s), if any, from the product. Examples of the known technique for condensation and deprotection include the methods as disclosed in the following literatures of (1) to (5):
(1) M. Bodanszky and M. A. Ondetti, Peptide Synthesis, Interscience Publishers, New York, 1966;
(2) Schroeder and Luebke, The Peptide, Academic Press, New York, 1965;
(3) Nobuo Izumiya et al., Fundamentals and Experiments of Peptide Synthesis, Maruzen, 1975;
(4) Haruaki Yajima and Shunpei Sakakibara, Biochemical Experiment Series 1, Protein Chemistry IV, 205, 1977; and
(5) Haruaki Yajima (ed.), Development of Drugs-Continued, Vol. 14, Peptide Synthesis, Hirokawa Shoten.

Further, after completion of the reaction, conventional purification processes such as solvent extraction, distillation, column chromatography, liquid chromatography, and recrystallization may be combined to purify and isolate the peptide fragment of the present invention. When the peptide fragment is obtained in a free form by the above process, it may be converted into a suitable salt by a known method. On the other hand, a salt obtained may be converted into a free form by a known method.

### [4] Polynucleotide Encoding NQO2

A polynucleotide encoding NQO2 may be any polynucleotide in so far as it contains a nucleotide sequence encoding the above NQO2 (DNA, RNA, or any chimera of DNA/RNA, preferably DNA). The polynucleotide may be NQO2-encoding DNA or RNA such as mRNA and may also be double-stranded or single-stranded. In case of a double-stranded structure, it may be double-stranded DNA, double-stranded RNA or a hybrid of DNA and RNA. In case of a single-strand structure, it may be a sense strand (or a coding strand) or an antisense strand (or a non-coding strand).

The quantity of mRNA of NQO2 may be determined using a polynucleotide encoding NQO2 by, for example, the known method disclosed in Jikken-Igaku (Experimental Medicine), Extra Issue, "New PCR and its applications," 15(7), 1997, or an analogous method.

The NQO2-encoding DNA may be any of genomic DNA, genomic DNA library, cDNA derived from the cells or tissues as mentioned above, cDNA library derived from the cells or tissues as mentioned above, and synthetic DNA. The vector for constructing the library may be any of bacteriophages, plasmids, cosmids, and phagemids. The total RNA fraction or a mRNA fraction prepared from the above cells or tissues may also be used for a direct amplification by Reverse Transcriptase Polymerase Chain Reaction (hereinafter abbreviated as RT-PCR method).

Specifically, the DNA encoding NQO2 may be any DNA containing the nucleotide sequence from the 176th to 868th bases of the nucleotide sequence of SEQ ID NO: 1 (hereinafter referred to as nucleotide sequence 1) or containing the nucleotide sequence from base 152nd to 844th bases of the nucleotide sequence of SEQ ID NO: 3 (hereinafter referred to as nucleotide sequence 3), or may be any DNA having a nucleotide sequence capable of hybridizing to nucleotide sequence 1 or 3 under high stringent conditions and encoding a protein having substantially the same activity in quality (e.g. reductase activity) as that of NQO2.

Examples of the DNA capable of hybridizing to nucleotide sequence 1 or 3 include DNA containing a nucleotide sequence having a homology of about 70% or more, preferably about 80% or more, more preferably about 90% or more, most preferably about 95% or more with nucleotide sequence 1 or 3.

The hybridization may performed by a per se known method or an analogous method, for example, the method disclosed in Molecular Cloning, 2nd Edition, J. Sambrook et al., Cold Spring Harbor Lab. Press, 1989. When a commercially available library is used, the hybridization can be performed according to the instructions attached thereto. The hybridization is more preferably performed under high stringent conditions.

The high stringent conditions include, for example, a sodium concentration of about 19 to 40 mM, preferably about 19 to 20 mM, and a temperature of about 50 to 70°C, preferably about 60 to 65°C. In particular, a sodium concentration of about 19 mM and a temperature of about 65°C are most preferred.

More specifically, as DNA encoding human-derived NQO2 containing the amino acid sequence represented by SEQ ID NO: 2, DNA containing nucleotide sequence 1, etc. can be used, and, as the DNA encoding mouse-derived NQO2 containing the amino acid sequence represented by SEQ ID NO: 4, DNA having nucleotide sequence 3, etc. can be used.

### [5] Antisense Nucleotide

A nucleotide containing a part of the nucleotide sequence of DNA encoding NQO2 or a part of a nucleotide sequence complementary to the DNA used herein includes not only DNA encoding a peptide fragment of NQO2 as described hereinafter, but also RNA.

A nucleotide containing a nucleotide sequence complementary to a target region of an objective nucleic acid, i.e., capable of hybridizing to the objective nucleic acid can be said to be "antisense" to the objective nucleic acid. On the other hand, a nucleotide containing a nucleotide sequence having a homology to a target region of an objective nucleic acid (i.e., nucleotide encoding a peptide fragment of a protein if the objective nucleic acid encodes the protein) can be said to be "sense" to the objective nucleic acid. The wording "corresponding" used herein means to have a homology to or to be complementary to a certain sequence of a nucleotide including a gene, a nucleotide sequence or a nucleic acid. The wording "to have a homology" or "to be complementary" means that the identity or complementation between nucleotide sequences is about 70% or more, preferably about 80% or more, more preferably about 90% or more, most preferably about 95% or more. Further, the "corresponding" between a nucleotide, a nucleotide sequence or a nucleic acid, and a peptide (protein) usually means that the peptide (protein) has an amino acid sequence which is translated from the sequence of the nucleotide (nucleic acid) or the sequence complementary thereto.

The antisense nucleotide (nucleic acid) of NQO2 gene can be designed and synthesized based on the nucleotide sequence information of a cloned or sequenced DNA encoding NQO2. Such a nucleotide (nucleic acid) can inhibit the replication or expression of NQO2 gene. For example, it can hybridize to RNA transcribed from NQO2 gene to inhibit the synthesis (processing) or function (translation into NQO2) of mRNA, or can regulate or control the expression of NQO2 gene through the interaction with NQO2-related RNA. The nucleotide complementary to a selected sequence of NQO2-related RNA, and the nucleotide capable of specifically hybridizing to NQO2-related RNA is useful for regulating or controlling the expression of NQO2 gene in vivo or in vitro or is useful for treating or diagnosing diseases or the like.

The target region of NQO2 gene for the antisense nucleotide may have any length without limitation in so far as it can hybridize to the antisense nucleotide so that the translation into NQO2 protein can be inhibited. The target region may be the entire sequence or a partial sequence of NQO2 mRNA and may be as short as about 15 bases or as long as the entire sequence of mRNA or an initial transcribed product. In view of easy synthesis and an antigenic problem, preferred examples include, but are not limited to, oligonucleotides of about 15 to about 30 bases. The target region may be selected from any regions within NQO2 gene and specifically be selected from 5'-end hairpin loop, 5'-end 6-base pair repeat, 5'-end untranslated region, polypeptide translation-initiation codon, protein-coding region, ORF translation-initiation codon, 3'-end untranslated region, 3'-end palindrome region, exon-intron boundary region, and 3'-end hairpin loop of NQO2 gene. Also, the target region may preferably be an intron part of NQO2 gene.

Further, NQO2 antisense nucleotide may be not only that hybridizable to NQO2 mRNA or an initial transcribed product of NQO2 gene to inhibit translation thereof into NQO2 protein, but also that capable of binding to NQO2 gene which is double-stranded DNA to form a triple-stranded (triplex) structure to inhibit the transcription of RNA.

Examples of the antisense nucleotide include a deoxynucleotide containing 2-deoxy-D-ribose, a deoxynucleotide containing D-ribose, other types of nucleotides which are N-glycosides of purine or pyrimidine base, other polymers having a non-nucleotide skeleton (such as a commercially available protein nucleic acid and synthetic sequence-specific nucleic acid polymer), and other polymer having a specific bond (such a polymer may contain a nucleotide having a configuration that allows the base pairing or the base attachment as found in DNA or RNA). Such antisense nucleotides may be double-stranded DNA, single-stranded DNA, double-stranded RNA, single-stranded RNA, or a DNA:RNA hybrid. Further, such an antisense nucleotide may be an unmodified polynucleotide (or unmodified oligonucleotide). Furthermore, it may be a modified polynucleotide in which a known modification is introduced, such as a polynucleotide having a label known in the field, a capped product, a methylated product, a polynucleotide in which one or more natural nucleotides are replaced with one or more analogues, a polynucleotide having intramolecular nucleotide modification such as one having a non-charged bond (e.g. methylphosphonate, phosphotriester, phosphoramidate, and carbamate), one having a charged bond or a sulfur-containing bond (e.g. phosphorothioate and phosphorodithioate), for example, one having a side chain group such as a protein (nuclease, nuclease inhibitor, toxin, antibody, signal peptide, poly-L-lysine, etc.) or a sugar (e.g. monosaccharide, etc.), one having an intercurrent compound (e.g. acridine, psoralen, etc.), one containing a chelate compound (e.g. metal, radioactive metal, boron, or oxidative metal, etc.), one containing an alkylating agent, and one having a modified bond (e.g. α anomer type nucleic acid, etc.). The "nucleoside", "nucleotide" and "nucleic acid" used herein may contain not only purine and pyrimidine bases, but also modified other heterocyclic bases. Such modified materials may contain methylated purine and pyrimidine, acylated purine and pyrimidine, or other heterocyclic rings. The modified nucleotide may also have a modified sugar moiety, for example, one or more hydroxyl groups may be substituted with one or more halogens or aliphatic groups or may be converted into other functional groups such as ether and amine.

The antisense nucleotide (nucleic acid) is RNA, DNA, or a modified nucleic acid (RNA or DNA). Examples of the modified nucleic acid include, but are not limited to, a sulfur derivative of a nucleic acid, a thiophosphate derivative of a nucleic acid, and a decomposition-resistant polynucleoside amide or oligonucleoside amide. The antisense nucleic acid of the present invention may preferably be designed based on the following principles. That is, the antisense nucleic acid should be more stable in cells; the cell permeability of the antisense nucleic acid should be more enhanced; the affinity for a target sense strand should be higher; and if the antisense nucleic acid has toxicity, such toxicity should be lowered.

Many modification techniques for such purposes are known in the art, for example, the techniques are disclosed in J. Kawakami et al., Pharm Tech Japan, Vol. 8, pp.247, 1992; Vol. 8, pp.395, 1992; S. T. Crooke et al., ed. Antisense Research and Applications, CRC Press, 1993; etc.

The antisense nucleic acid may contain an altered or modified sugar, base or linkage, may be provided in a special form such as liposomes, microspheres, etc., may be applicable to gene therapy, or may be provided in an added form. Examples of such an added form include a polycation such as polylysine that serves to neutralize the charge of a phosphate backbone, and a hydrophobic material such as lipid (e.g. phospholipid, cholesterol, etc.) that enhances the interaction with cell membranes or increase the uptake of the nucleic acid. Examples of the preferred lipid to be added include cholesterol and derivatives thereof (e.g. cholesteryl chloroformate, cholic acid, etc.). These materials may be attached to the 3' or 5' end of the nucleic acid or may be attached through a base, a sugar, or an intramolecular nucleoside linkage. Another moiety may include a group for capping that is specifically placed at the 3' or 5' end of the nucleic acid to prevent degradation by a nuclease such as exonuclease and RNase. Examples of the group for capping include, but are not limited to, hydroxyl-protecting groups known in the art field such as glycols, e.g., polyethylene glycol and tetraethylene glycol.

The antisense nucleotide of the present invention may also include a ribozyme that can specifically cleave NQO2 mRNA or an initial transcribed product of the NQO2 gene at a site inside of the coding region (including an intron part in case of an initial transcribed product). The "ribozyme" refers to RNA having a nucleic acid-cleaving enzyme activity. Recently, however, it has been found that certain oligo-DNA having a nucleotide sequence corresponding to the enzyme activity also exhibits the nucleic acid-cleaving activity. Therefore, the wording "ribozyme" used herein refers to not only the RNA but also the DNA that has a sequence-specific nucleic acid-cleaving activity. Examples of the most general ribozyme include self-splicing RNA such as that found in infectious RNA of viroids or virusoids, and a hammer head type and a hairpin type are known. The hammer head type can exhibit the enzyme activity when having about 40 bases, and can specifically cleave only a target mRNA by constituting several bases adjacent to each of both ends of the hammer head-structured part (totally about 10 bases) with a sequence complementary to the target site of the mRNA. This type of ribozyme uses only RNA as the substrate and therefore has an additional advantage that it does not attack the genome DNA. When NQO2 mRNA itself has a double-stranded structure, the target sequence may be converted into a single-stranded structure by using a hybrid ribozyme to which a virus nucleic acid-derived RNA motif capable of specifically binding to RNA helicase is coupled (Proc. Natl. Acad. Sci. USA, 98(10), 5572-5577, 2001). Further, when the ribozyme is used in the form of an expression vector containing DNA encoding it, the ribozyme may also be a hybrid ribozyme to which a sequence modified from tRNA is further coupled to enhance the transfer of a transcribed product into a cytoplasm (Nucleic Acids Res., 29(13), 2780-2788, 2001).

The antisense nucleotide of the present invention may also include a double-stranded oligo-RNA complementary to a partial sequence of the coding region (containing an intron part in case of an initial transcript) of NQO2 mRNA or an initial transcribed product of the NQO2 gene. So-called RNA interference (RNAi) phenomenon, wherein the introduction of a short double-stranded RNA into cells leads to degradation of mRNA complementary to the introduced RNA, has already been known in nematodes, insects, plants, and the like. Recently, this phenomenon has also been found in mammalian cells (Nature, 411(6836), 494-498, 2001) and has been noted as an alternative technique of a ribozyme.

The antisense oligonucleotide and the ribozyme of the present invention can be prepared by determining a target region of mRNA or an initial transcribed product based on the information about cDNA sequence or the genomic DNA sequence of NQO2 and synthesizing a sequence complementary to the target region with a commercially available DNA/RNA automatic synthesizer (for example, Applied Biosystems, Beckman, etc.). The double-stranded oligo-RNA having RNAi activity can be prepared by synthesizing a sense strand and an antisense strand with a DNA/RNA automatic synthesizer, respectively, denaturing them in an appropriate annealing buffer solution, for example, at a temperature of about 90 to about 95°C for about one minute, and then annealing them at a temperature of about 30 to about 70°C for about one to about eight hours. Alternatively, a longer double-stranded polynucleotide can be prepared by synthesizing complementary oligonucleotide strands in an alternately overlapped manner, annealing them and then subjecting them to ligation with a ligase.

NQO2 expression-inhibiting activity of the antisense nucleic acid of the present invention can be examined using a transformant containing DNA encoding NQO2, an in vivo or in vitro expression system for NQO2 gene, or an in vivo or in vitro translation system for NQO2. The nucleic acid itself may be introduced into cells according to various known methods.

### [6] DNA Encoding Peptide Fragment of NQO2

DNA encoding a peptide fragment of NQO2 may be any DNA in so far as it contains the above nucleotide sequence encoding the peptide fragment of NQO2. DNA encoding a peptide fragment of NQO2 may also be any of genomic DNA, genomic DNA library, cDNA derived from the cells or tissues as mentioned above, cDNA library derived from the cells or tissues as mentioned above, and synthetic DNA. The vector to be used for the library may be any of bacteriophages, plasmids, cosmids, and phagemids. A mRNA fraction prepared from the above cells or tissues may also be amplified directly by using Reverse Transcriptase Polymerase Chain Reaction (hereinafter abbreviated as RT-PCR method).

Examples of DNA encoding a peptide fragment of NQO2 include (1) DNA having a partial nucleotide sequence of nucleotide sequence 1 or 3 and (2) DNA having a partial nucleotide of DNA encoding NQO2 which has a nucleotide sequence capable of hybridizing to nucleotide sequence 1 or 3 under high stringent conditions and has substantially the same activity in quality (such as a reductase activity) as that of NQO2 peptide.

Examples of DNA capable of hybridizing to nucleotide sequence 1 or 3 under high stringent conditions include DNA having a homology of about 70% or more, preferably about 80% ore more, more preferably about 90% or more, most preferably about 95% or more with nucleotide sequence 1 or 3.

### [7] Process for Producing DNA Encoding NQO2 or Its Peptide Fragment

DNA fully encoding NQO2 or its peptide fragment (hereinafter also collectively referred to as NQO2 in some cases) may be cloned by PCR amplification using synthetic DNA primers each having a partial nucleotide sequence of NQO2 or by hybridizing DNA integrated in a suitable vector to one labeled with a DNA fragment or synthetic DNA encoding a part or full region of NQO2 to select the desired DNA. The hybridization may be carried out by, for example, the method disclosed in Molecular Cloning, 2nd (J. Sambrook et al., Cold Spring Harbor Lab. Press, 1989). A commercially available library may also be used according to the instructions attached thereto.

The conversion of a nucleotide sequence of DNA may be carried out by a per se known method such as ODA-LA PCR method, Gupped duplex method, Kunkel method, etc. or an analogous method, with PCR or a known kit such as Mutan™-super Express KmG (Takara Shuzou Co., Ltd) and Mutan™-K (Takara Shuzou Co., Ltd).

Depending on a particular purpose, the desired cloned DNA encoding NQO2 may be used as it is or, if desired, it may be used after digestion with restriction enzyme(s) or addition of a linker. The DNA may have ATG as a translation initiation codon at the 5' end-side and TAA, TGA, or .TAG as a translation termination codon at the 3' end-side. The translation initiation and termination codons may be added with a suitable synthetic DNA adapter.

### [8] Expression Vector

An expression vector of NQO2 can be produced by, for example, (a) cutting out the desired DNA fragment from DNA encoding NQO2 and (b) ligating the DNA fragment into a suitable expression vector downstream from a promoter.

Examples of the vector include plasmids derived from *Escherichia coli* (e.g. pBR322, pBR325, pUC12, and pUC13); plasmids derived from *Bacillus subtilis* (e.g. pUB110, pTP5 and pC194); plasmids derived from yeast (e.g. pSH19 and pSH15); bacteriophages such as λ-phage; animal viruses such as retrovirus, vaccinia virus and baculovirus; and other vectors such as pA1-11, pXT1, pRc/CMV, pRc/RSV, pcDNAI/Neo, etc.

Any promoter may be used in so far as it is suitable for a host for use in the gene expression. In case of using animal cells as a host, examples of the promoter include SRα promoter, SV40 promoter, LTR promoter, CMV promoter, HSV-TK promoter and the like.

Among them, preferably, CMV promoter and SRα promoter are used. When the host is bacteria of the genus *Escherichia,* the promoter is preferably trp promoter, lac promoter, recA promoter, λP_{L} promoter, lpp promoter, or the like. When the host is bacteria of the genus *Bacillus,* the promoter is preferably SPO1 promoter, SPO2 promoter, penP promoter, or the like. When the host is yeast, the promoter is preferably PHO5 promoter, PGK promoter, GAP promoter, ADH promoter, or the like. When the host is insect cells, the promoter is preferably polyhedrin promoter, P10 promoter, or the like.

If desired, the expression vector may further contains an enhancer, a splicing signal, a poly A addition signal, a selection marker, an SV40 duplicate origin (hereinafter abbreviated to SV40ori in some cases), etc. Examples of the selection marker include a dihydrofolic acid reductase (hereinafter abbreviated to dhfr in some cases) gene (methotrexate (MTX) resistant), an ampicillin-resistant gene (hereinafter abbreviated to Amp^{r} in some cases), a neomycin-resistant gene (hereinafter abbreviated to Neo^{r} in some cases, G418 resistant), etc. Particularly, when CHO (dhfr⁻) cells are used together with dhfr gene as a selection marker, the desired gene may also be selected using a thymidine-free medium.

Further, if necessary, a signal sequence suitable for a host may be added to the N-terminal side of NQO2. A PhoA signal sequence, an OmpA signal sequence, or the like in case that the host is bacteria of the genus *Escherichia;* an α-amylase signal sequence, a subtilisin signal sequence, or the like in case that the host is bacteria of the genus *Bacillus;* an MFα signal sequence, an SUC2 signal sequence, or the like in case that the host is yeast; an insulin signal sequence, an α-interferon signal sequence, an antibody molecule signal sequence, or the like, in case that the host is animal cells can be used, respectively.

### [9] Transformant

A transformant may be produced by using thus-constructed vector containing DNA coding for NQO2.

Examples of the host include bacteria of the genus *Escherichia,* bacteria of the genus *Bacillus,* yeast, insect cells, insects, and animal cells.

Specific examples of bacteria of the genus *Escherichia* include *Escherichia coli* K12·DH1 (Proceedings of the National Academy of Sciences of USA, Vol. 60, 160, 1968), JM103 (Nucleic Acids Research, Vol. 9, 309, 1981), JA221 (Journal of Molecular Biology, Vol. 120, 517, 1978), HB101 (Journal of molecular Biology, Vol. 41, 459, 1969), and C600 (Genetics, Vol. 39, 440, 1954).

Examples of bacteria of the genus *Bacillus* include *Bacillus subtilis* MI114 (Gene, Vol. 24, 255, 1983) and 207-221 (Journal of Biochemistry, Vol. 95, 87, 1984).

Examples of yeast include *Saccharomyces cerevisiae* AH22, AH22R⁻, NA87-11A, DKD-5D and 20B-12; *Schizosaccharomyces pombe* NCYC1913 and NCYC2036; and *Pichia pastoris*.

Examples of insect cells include a *Spodoptera frugiperda* cells (Sf cells), MG1 cells derived from the midgut of *Trichoplusia ni*, High Five™ cells derived from the egg of *Trichoplusia ni, Mamestra brassicae*-derived cells, and *Estigmena acrea*-derived cell. When the virus is BmNPV, *Bombyx mori* N cells (BmN cells) can be used. As the Sf cells, for example, Sf9 cells (ATCC CRL1711) and Sf21 cells (both, Vaughn J. L. et al., In Vivo, 13, 213-217, 1977) can be used.

As the insects, for example, a silkworm larva can be used (Maeda et al., Nature, Vol. 315, 592, 1985).

Examples of the animal cells include monkey cell COS-7, Vero, Chinese hamster cell CHO (hereinafter abbreviated to CHO cells), dhfr gene-deficient Chinese hamster cell CHO (hereinafter abbreviated to CHO(dhfr⁻) cells), mouse L cells, mouse AtT-20 cells, mouse myeloma cells, rat GH3 cells, and human FL cells.

For example, transformation of bacteria of the genus *Escherichia* can be performed according to the method disclosed in Proceedings of the National Academy of Sciences of USA, Vol. 69, 2110, 1972; and Gene, Vol. 17, 107, 1982.

For example, transformation of bacteria of the genus *Bacillus* can be performed according to the method disclosed in Molecular & General Genetics, Vol. 168, 111, 1979.

For example, transformation of yeast can be performed according to the method disclosed in Methods in Enzymology, Vol. 194, 182-187, 1991; and Proc. Natl. Acad. Sci. USA, Vol. 75, 1929, 1978.

For example, transformation of insect cells or an insect can be performed according to the method disclosed in Bio/Technology, 6, 47-55, 1988.

For example, transformation of animal cells can be performed by the method disclosed in Cell Engineering, Separate Vol. 8, New Cell Engineering Experiment Protocol, 263-267, 1995, Shujun Company; and Virology, Vol. 52, 456, 1973.

Thus, the transformant transformed with the expression vector containing DNA encoding NQO2 is obtained.

### [10] Process for Producing Recombinant NQO2

When the transformant whose host is bacteria of the genus *Escherichia* or bacteria of the genus *Bacillus* is cultured, a liquid culture medium is a suitable medium to be used for the culture. Such a medium contains a carbon source, a nitrogen source, inorganic materials, and other materials, which are required for the growth of the transformant. Examples of the carbon source include glucose, dextrin, soluble starch, and sucrose. Examples of the nitrogen source include organic or inorganic substances such as ammonium salts, nitrates, corn steep liquor, peptone, casein, meat extract, soybean-cake, and potato extract. As the inorganic substance, for example, there are calcium chloride, sodium dihydrogen phosphate, and magnesium chloride. Yeast extract, vitamins, or growth-promoting factors, etc., may also be added. The medium preferably has a pH of about 5 to 8.

As a culture medium for culturing bacteria of the genus *Escherichia*, for example, M9 medium which contains glucose and casamino acid (Miller, Journal of Experiments in Molecular Genetics, 431-433, Cold Spring Harbor Laboratory, New York, 1972) is preferred. If necessary, a drug such as 3β-indolyl acrylic acid can be added to the medium to improve the efficiency of the promoter.

When the host is bacteria of the genus Escherichia, usually, the culture is performed at a temperature of about 15 to 43°C for about 3 to 24 hours. If necessary, aeration or stirring may be applied.

When the host is bacteria of the genus *Bacillus,* usually, the culture is performed at a temperature of about 30 to 40°C for about 6 to 24 hours. If necessary, aeration or stirring may also be applied.

When the transformant whose host is yeast is cultured, for example, the culture medium may be Burkholder minimum medium (Bostian, K. L. et al., Proceedings of the National Academy of Sciences of USA, Vol. 77, 4505, 1980) or SD medium containing 0.5% casamino acid (Bitter, G. A. et al., Proceedings of the National Academy of Sciences of USA, Vol. 81, 5330, 1984). The pH of the medium is preferably adjusted to about 5 to 8. Usually, the culture is performed at a temperature of about 20°C to 35°C for about 24 to 72 hours. If necessary, aeration or stirring may be applied.

When the transformant whose host is insect cells or insect is cultured, for example, the culture medium may be Grace's Insect Medium appropriately supplemented with an additive such as inactivated 10% bovine serum (Grace, T. C. C., Nature, 195, 788, 1962). It is preferred to adjust the pH of the culture medium to about 6.2 to 6.4. Usually, the culture is performed at about 27°C for about three to five days. If necessary, aeration or stirring may be applied.

When the transformant whose host is animal cells is cultured, for example, the culture medium may be about 5 to 20% fetal bovine serum-containing MEM medium (Science, Vol. 122, 501, 1952), DMEM medium (Virology, Vol. 8, 396, 1959), RPMI 1640 medium (Journal of the American Medical Association, Vol. 199, 519, 1967), or 199 medium (Proceeding of the Society of the Biological Medicine, Vol. 73, 1, 1950). The pH is preferably from about 6 to 8. Usually, the culture is performed at a temperature of about 30°C to 40°C for about 15 to 60 hours. If necessary, aeration or stirring may be applied.

According to the above methods, NQO2 can be produced inside the transformant cells, at the cell membranes of the transformant, or outside the transformant cells.

For separating and purifying NQO2 from the above culture, for example, the following methods can be performed.

When NQO2 is extracted from cultured microbial cells or cells, for example, the following method is appropriately used. That is, after completion of the culture, the microbial cells or cells are collected by a known method and they are suspended in a suitable buffer. Then, the microbial cells or cells are ruptured by ultrasonic wave, lysozyme and/or freeze-thaw, followed by centrifugation or filtration to obtain a crude NQO2 extract. The buffer solution may contain a protein-denaturing agent such as urea and guanidine hydrochloride and a surfactant such as Triton X-100™. When NQO2 is secreted into a culture solution, the supernatant is separated from the microbial cells or cells after completion of the culture by a known method and the supernatant is collected.

Purification of NQO2 contained in the culture supernatant thus obtained or an extract can be performed by suitably combining per se known methods for separation and purification techniques. Examples of such known separation and purification techniques include solubility difference-based methods such as salting-out and solvent precipitation method; molecular weight difference-based methods such as dialysis, ultrafiltration, gel filtration, and SDS-polyacrylamide gel electrophoresis; electrical charge difference-based methods such as ion exchange chromatography; specific affinity-based methods such as affinity chromatography; hydrophobic difference-based methods such as reverse phase high performance liquid chromatography; and isoelectric point difference-based methods such as isoelectric focusing.

When NQO2 thus obtained is in a free form, it may be converted into a salt by a known method or an analogous method. On the other hand, when NQO2 obtained is in a salt form, it may be converted into a free form or another salt by a known method or an analogous method.

NQO2 produced by a transformant may be subjected to treatment with a suitable protein-modifying enzyme before or after purification to modify it in the desired manner or to partly remove a polypeptide fragment. Examples of the protein-modifying enzyme include trypsin, chymotrypsin, arginyl endopeptidase, protein kinase, and glycosidase.

The activity of the resulting NQO2 or a salt thereof can be measured by binding assay with a labeled ligand or by enzyme immunoassay using a specific antibody.

### [11] Anti-NQO2 Antibody

An antibody against NQO2, its peptide fragment or a salt thereof may be either of polyclonal and monoclonal antibodies in so far as it can recognize NQO2, its peptide fragment or a salt thereof. In particular, the antibody capable of neutralizing reductase activity of NQO2, its peptide fragment or a salt thereof is preferably used.

The antibody against NQO2, its peptide fragment or a salt thereof (hereinafter abbreviated to NQO2, etc. in some cases) can be produced using NQO2, etc. as an antigen according to a per se known method for producing antibodies or antisera.

### [Preparation of Monoclonal Antibody]

### (a) Preparation of Monoclonal Antibody-Producer Cells

NQO2, etc. are administered alone or in combination with a carrier or diluent to a mammal at a site capable of producing the antibody. In order to enhance the capability to produce the antibody, a complete or incomplete Freund's adjuvant may also be administered. Usually, the administration is performed once every 2 to 6 weeks and 2 to 10 times in total. Examples of the applicable mammals include monkey, rabbit, dog, guinea pig, mouse, rat, sheep, and goat. Mouse and rat are preferably used.

When producing monoclonal antibody-producer cells, a monoclonal antibody-producer hybridoma can be prepared by selecting a warm-blooded animal immunized with an antigen, for example, an individual having a detectable antibody titer from mice, collecting its spleen or lymph node after two to five days from the final immunization and then subjecting the antibody-producer cells contained therein to fusion with myeloma cells. The measurement of antibody titer in an antiserum can be performed, for example, by reacting a labeled NQO2, etc. as described hereinafter with the antiserum, and then measuring the activity of the labeling agent bound to the antibody. The cell fusion can be performed according to a known method, for example, the method of Koehler and Milstein (Nature, Vol. 256, 495, 1975). As a fusion accelerator, for example, there are polyethylene glycol (PEG) and Sendai virus. PEG is preferably used.

Examples of the myeloma cells include NS-1, P3U1, and SP2/0, and P3U1 is preferably used. The ratio of the number of the antibody-producer cells used (spleen cells) to the number of the myeloma cells is preferably from about 1:1 to about 20:1. The cell fusion can efficiently be performed by adding PEG (preferably PEG 1000 to PEG 6000) at a concentration of about 10 to 80% and incubating at a temperature of about 20 to 40°C, preferably about 30 to 37°C, for about 1 to 10 minutes.

Various methods can be used for screening the monoclonal antibody-producer hybridoma. For example, there are a method comprising adding a supernatant of a hybridoma culture to a solid phase (e.g. a microplate) onto which an antigen of NQO2, etc. is adsorbed directly or with a carrier, and then adding an anti-immunoglobulin antibody (anti-mouse immunoglobulin antibody in case that mouse cells are used for the cell fusion) labeled with a radioactive substance, an enzyme or the like, or adding protein A to detect the monoclonal antibody bound to the solid phase; and a method comprising adding a supernatant of a hybridoma culture to a solid phase onto which an anti-immunoglobulin antibody or protein A is adsorbed, and adding NQO2, etc. labeled with a radioactive substance or an enzyme to detect the monoclonal antibody bound to the solid phase.

Selection of the monoclonal antibody may be performed by a per se known method or an analogous method. Usually, it is performed by using a medium for animal cells to which HAT (hypoxanthine, aminopterin and thymidine) has been added, or the like. As a medium for selection and breeding, any medium in which the hybridoma can grow can be used. Examples of the medium to be used include RPMI 1640 medium containing 1 to 20%, preferably 10 to 20% of fetal calf serum, GIT medium (Wako Pure Chemicals Industries, Ltd.) containing 1 to 10% of fetal calf serum, and a serum-free medium for hybridoma culture (SFM-101, Nissui Pharmaceutical Co., Ltd.), etc. Usually, the culture temperature is from 20 to 40°C, preferably about 37°C. The culture time is generally from five days to three weeks, preferably one to two weeks. The culture is usually performed in 5% carbon dioxide gas. The antibody titer of a supernatant of a hybridoma culture may be determined in the same manner as that for the above measurement of the antibody titer in the antiserum.

### (b) Purification of Monoclonal Antibody

The separation and purification of the monoclonal antibody can be performed according to the separation and purification of normal immunoglobulins [e.g. salting-out, precipitation with alcohol, isoelectric precipitation, electrophoresis, adsorption and desorption with ion exchangers (e.g. DEAE), ultracentrifugation, gel filtration, and a specific purification method in which only the antibody is collected onto an active adsorbent such as an antigen-binding solid phase, protein A or protein G and then the antibody is obtained by dissociating the bond.

### [Preparation of Polyclonal Antibody]

The polyclonal antibody of the present invention can be produced by a per se known method or an analogous method. For example, it can be prepared by forming a conjugate of an immunogen (antigen of NQO2, etc.) and a carrier protein, immunizing a mammal in the same manner as that described for the production of the monoclonal antibody, collecting a fraction containing an antibody to NQO2, etc. from the immunized animal, and separating and purifying the antibody.

Regarding the immunogen-carrier protein conjugate for use in the immunization of the mammal, any kind of the carrier protein and any mixing ratio of the carrier and a hapten may be used in so far as an antibody against the hapten crosslinked to the carrier and used for immunization is efficiently produced. For example, in a method to be employed, bovine serum albumin, bovine thyroglobulin, Keyhole-limpet hemocyanin, or the like is coupled to a hapten in a weight ratio of about 0.1 to 20, preferably about 1 to 5, relative to the hapten 1.

A variety of condensing agents can be used for the coupling between the hapten and the carrier. For example, glutaraldehyde, carbodiimide, a maleimide active ester, or a thiol or dithiopyridyl group-containing active ester reagent can be used.

The condensation reaction product is administered alone or in combination with a carrier or diluent to a warm-blooded animal at a site capable of producing an antibody. In order to enhance the capability to produce the antibody, a complete or incomplete Freund's adjuvant may also be administered. Usually, the administration is performed once every 2 to 6 weeks and 3 to 10 times in total.

The polyclonal antibody can be collected from blood, ascites fluid, or the like, preferably from blood of the mammal immunized by the above method.

The polyclonal antibody titer in the antiserum can be determined in the same manner as the above determination of the monoclonal antibody titer. The separation and purification of the polyclonal antibody can be performed by the same method as that described for the separation and purification of the monoclonal antibody.

### [12] Method and Kit for Screening Candidate Compounds for Medicine for Bone diseases

A compound or a salt thereof that inhibits the reductase activity of NQO2, its peptide fragment or a salt thereof has osteogenesis-promoting action, osteoblast (including a precursor osteoblast) differentiation-inducing action, osteoblast (including a precursor osteoblast) differentiation induction-promoting action, chondrogenesis-promoting action, chondrocyte (including a precursor chondrocyte) differentiation-inducing action, chondrocyte (including a precursor chondrocyte) differentiation induction-promoting action, BMP action-enhancing action, etc. Therefore, the compound or a salt thereof is useful as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent.

Further, the compound or a salt thereof can also be used as an agent for preventing or treating bone diseases. More specifically, the compound or a salt thereof can be used as an agent for preventing or treating non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; or articular diseases typically including cartilage diseases such as osteoarthritis and rheumatoid arthritis.

Furthermore, the compound or a salt thereof can also be used as a bone tissue-repairing agent after a surgery of multiple myeloma, lung cancer, breast cancer, or the like. In the field of dentistry, the compound or a salt thereof may also be used for treatment of periodontosis, repair of a periodontosis-induced periodontium defect, stabilization of an artificial root of a tooth, residual ridge formation, repair of cleft palate, or the like. Therefore, NQO2, its peptide fragment or a salt thereof (hereinafter also referred to as NQO2, etc.) is useful as a reagent for screening a compound or a salt thereof that inhibits the activity (e.g. the reductase activity, etc.) of NQO2, etc..

On the other hand, the compound or a salt thereof that promotes the reductase activity of NQO2 has osteogenesis-inhibiting action, osteoblast (including a precursor osteoblast) differentiation-inhibiting action, osteoblast (including a precursor osteoblast) differentiation induction-inhibiting action, chondrogenesis-inhibiting action, chondrocyte (including a precursor chondrocyte) differentiation-inhibiting action, chondrocyte (including a precursor chondrocyte) differentiation induction-inhibiting action, or BMP action-inhibiting action. Therefore, the compound or a salt thereof is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

Moreover, the compound or a salt thereof can also be used as an agent for preventing or treating bone diseases such as osteopetrosis and osteochondroma. Thus, NQO2, etc. is useful as a reagent for screening a compound or a salt thereof that promotes the reductase activity of NQO2, etc..

Therefore, the present invention also provides:
(1) a method for screening a compound or a salt thereof that promotes the reductase activity of NQO2, etc. (hereinafter also referred to as a promoting drug in some cases) or a compound that inhibits the reductase activity of NQO2, etc. (hereinafter also referred to as a inhibiting drug in some cases); and more specifically
(2) a method for screening a promoting drug or inhibiting drug, which comprises comparing (i) a case where NQO2, etc. is brought into contact with a substrate with (ii) a case where NQO2, etc. is brought into contact with the substrate and a test compound.

Specifically, in the screening method, the reductase activities of NQO2, etc. in the cases (i) and (ii) are measured and compared.

The screening method of the present invention will be illustrated more specifically hereinafter.

The substrate may be any substance in so far as it can be used as a substrate for NQO2, its peptide fragment or a salt thereof. For example, dichlorophenol-indophenol, menadione, methyl red, etc., can be used.

As the test compound, for example, there are peptides, proteins, non-peptidic compounds, synthetic compounds, fermentation products, cell extracts, plant extracts, animal tissue extracts and the like. These compounds may be novel compounds or known compounds.

For performing the above screening method, NQO2, etc. are suspended in a buffer suitable for the screening to prepare a standard sample of NQO2, etc. Any buffer can be used in so far as it does not interfere with binding between NQO2, etc. and the substrate, for example, there are a phosphate buffer or a Tris-HCl buffer with a pH of about 4 to 10 (preferably about 6 to 8).

The reductase activity of NQO2, etc. can be measured by a per se known method, for example, the method disclosed in the report by Kebin Wu et al. (Archives of Biochemistry and Biophysics, Vol. 347, pp221-228, 1997) or an analogous method.

For example, a test compound that decreases the reductase activity of NQO2, etc. by about 20% or more, preferably 50% or more in the case (ii) as compared with the case (i) may be selected as a reductase activity-inhibiting compound. On the other hand, a test compound that increases the reductase activity of NQO2, etc. by about 20% or more, preferably 50% or more in the case (ii) as compared with the case (i) may be selected as a reductase activity-promoting compound.

The screening kit of the present invention comprises NQO2, its peptide fragment thereof, or a salt thereof. As an example of the screening kit of the present invention, there is the following kit:

### [Screening Reagents]

### (1) Buffer for Measurement

Tris-HCl buffer at a pH of 7.4 (containing sodium chloride)

### (2) Protein Standard Sample

NQO2

### (3) Substrate

25 µM dichlorophenol indophenol

### (4) Electron Donor

50 µM NRH (dihydronicotinamide riboside)

### Detection

reduction in absorbance at 600 nm

### [Measurement Method]

NRH (or another electron donor) and the substrate, and further a test compound are added to NQO2, etc. and the reaction is performed at 25°C. After completion of the reaction, a reduction in absorbance at 600 nm is measured to determine the activity.

The compound or a salt thereof obtained by using the screening method or kit of the present invention is that selected from the above-mentioned test compounds, and promoting or inhibiting the reductase activity of NQO2, etc.

As the salt of the compound, particularly, a physiological acceptable acid-addition salt is preferred. Examples of the salt to be used include a salt with an inorganic acid (such as hydrochloric acid, phosphoric acid, hydrobromic acid or sulfuric acid) and a salt with an organic acid (such as acetic acid, formic acid, propionic acid, fumaric acid, maleic acid, succinic acid, tartaric acid, citric acid, malic acid, oxalic acid, benzoic acid, methanesulfonic acid or benzenesulfonic acid) and the like.

### [13] Use of NQO2 Activity-Inhibiting or Promoting Agent

A compound that inhibits the reductase activity of NQO2, etc. is useful, for example, as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent.

Further, for example, the compound is also useful as an agent for preventing or treating bone diseases. More specifically, the compound is useful as a safe and less toxic agent for preventing or treating various bone diseases, for example, non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; or articular diseases typically including cartilage diseases such as osteoarthritis and rheumatoid arthritis.

Furthermore, the compound can also be used as a bone tissue-repairing agent after a surgery of multiple myeloma, lung cancer, breast cancer, or the like. In the field of dentistry, such a compound can also be used for treatment of periodontosis, repair of a periodontosis-induced periodontium defect, stabilization of an artificial root of a tooth, residual ridge formation, repair of cleft palate, or the like.

On the other hand, a compound that promotes the activity of NQO2, etc. has osteogenesis-inhibiting action, osteoblast (including a precursor osteoblast) differentiation-inhibiting action, osteoblast (including a precursor osteoblast) differentiation induction-inhibiting action, chondrogenesis-inhibiting action, chondrocyte (including a precursor chondrocyte) differentiation-inhibiting action, chondrocyte (including a precursor chondrocyte) differentiation induction-inhibiting action, BMP action-inhibiting action, etc. Therefore, the compound is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

Further, the compound is also useful as a safe and less toxic agent for preventing or treating bone diseases such as osteopetrosis and osteochondroma.

When the compound or a salt thereof obtained by using the screening method or kit of the present invention is used as the above-mentioned pharmaceutical composition, it can be performed according to a conventional method. For example, the compound may be used orally in the form of a tablet, if necessary, coated with sugar, a capsule, an elixir, microcapsules, or the like, or parenterally in the form of an injectable preparation such as an aseptic solution or suspension in water or another pharmaceutically acceptable liquid. For example, the compound may be mixed with a known physiologically acceptable carrier, flavoring agent, excipient, vehicle, antiseptic, stabilizer, binder, and the like to form a preparation in a generally acceptable unit dosage form. The content of the active ingredient in the preparation may be set so as to provide an appropriate dose within a specified range.

Examples of the additive which may be mixed into a tablet, a capsule or the like include a binder such as gelatin, corn starch, tragacanth, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage form is a capsule, a liquid carrier such as fats and oils may further be added in addition to the above materials. A sterile composition for injection may be formulated according to a conventional preparation procedure, for example, by dissolving or suspending the active substance, a naturally occurring vegetable oil such as sesame oil, coconut oil, and the like in a vehicle such as water for injection. Examples of the aqueous liquid for injection include a physiological saline and an isotonic solution containing glucose or other auxiliary agents (e.g. D-sorbitol, D-mannitol and sodium chloride). The aqueous liquid may be used in combination with an appropriate dissolution aid, for example an alcohol (e.g. ethanol), a polyalcohol (e.g. propylene glycol and polyethylene glycol), and a nonionic surfactant (e.g. polysorbate 80™ and HCO-50). Examples of the oily liquid include sesame oil and soybean oil. The oily liquid may be used in combination with a dissolution aid such as benzyl benzoate and benzyl alcohol.

The above prophylactic and/or therapeutic agent may also be formulated with a buffer (e.g. a phosphate buffer and a sodium acetate buffer), a soothing agent (e.g. benzalkonium chloride and procaine hydrochloride), a stabilizer (e.g. human serum albumin and polyethylene glycol), a preservative (e.g. benzyl alcohol and phenol), an antioxidant, or the like. Usually, the resulting injectable preparation is filled in an appropriate ampoule.

The preparation thus obtained is safe and less toxic and therefore can be administered to human or mammals (e.g. rat, mouse, rabbit, sheep, pig, bovine, cat, dog, and monkey).

The dose of the compound or a salt thereof may vary depending on a particular subject, subject organ, symptom, way of administration, and the like. However, in general, for example, the NQO2 inhibitor may be orally administered to a patient with osteoporosis (60 kg in weight) in a dose of about 0.1 to 100 mg per day, preferably about 1.0 to 50 mg per day, more preferably about 1.0 to 20 mg per day. In case of parenteral administration, the NQO2 inhibitor may be, for example, in the form of an injectable preparation, to a patient with osteoporosis (60 kg in weight), usually, in a dose of about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [14] Use of Antisense Nucleotide

A nucleotide having a nucleotide sequence complementary to the polynucleotide encoding the above NQO2 or having a part thereof (i.e. the antisense nucleotide) can inhibit the expression of NQO2 and therefore is useful as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent, similarly to the above NQO2 activity inhibitor.

Further the antisense nucleotide is also useful as an agent, for example, for preventing or treating bone diseases. More specifically, the antisense nucleotide is useful as a safe and less toxic agent for preventing or treating a variety of bone diseases such as non-metabolic bone diseases in the field of orthopedics, for example, bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; and articular diseases typically including cartilage diseases such as osteoarthritis and rheumatoid arthritis. Further, the antisense nucleotide can also be used as a bone tissue-repairing agent after a surgery of multiple myeloma, lung cancer, breast cancer, or the like. In the field of dentistry, the antisense nucleotide can also be used for treatment of periodontosis, repair of a periodontosis-induced periodontium defect, stabilization of an artificial root of a tooth, residual ridge formation, repair of cleft palate, or the like.

When the antisense nucleotide is used as the above prophylactic or therapeutic agent, the nucleotide may be used alone or inserted into a suitable vector such as a retrovirus vector, an adenovirus vector and an adenovirus-associated virus vector before use according to a conventional manner. The nucleotide may be administered as it is or with a supplemental agent for enhancing uptake by using a gene gun or a catheter such as a hydrogel catheter.

For example, the nucleotide may be used orally in the form of a tablet, if necessary coated with sugar, a capsule, an elixir, microcapsules, or the like, or parenterally in the form of an injectable preparation such as an aseptic solution or suspension in water or another pharmaceutically acceptable liquid. For example, the nucleotide may be mixed with a known physiologically acceptable carrier, flavoring agent, excipient, vehicle, antiseptic, stabilizer, binder, or the like to form a preparation in a generally acceptable unit dosage form. The content of the active ingredient in such a preparation may be set so as to provide an appropriate dose within a specified range.

Examples of the additive which may be mixed into a tablet, a capsule or the like include a binder such as gelatin, corn starch, tragacanth, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage form is a capsule, a liquid carrier such as fats and oils may further be added to in addition to the above materials. A sterile composition for injection may be formulated according to a conventional preparation procedure, for example, by dissolving or suspending the active substance, a naturally occurring vegetable oil such as sesame oil and coconut oil, and the like in a vehicle such as water for injection. Examples of the aqueous liquid for injection include a physiological saline and an isotonic solution containing glucose or another auxiliary agent (such as D-sorbitol, D-mannitol and sodium chloride). Such an aqueous liquid may be used in combination with an appropriate dissolution aid, for example an alcohol (such as ethanol), a polyalcohol (such as propylene glycol and polyethylene glycol), and a nonionic surfactant (such as polysorbate 80™ and HCO-50). Examples of the oily liquid include sesame oil and soybean oil. The oily liquid may be used in combination with a dissolution aid such as benzyl benzoate and benzyl alcohol.

Further, the above prophylactic or therapeutic agent may also be formulated with a buffer (e.g. a phosphate buffer and a sodium acetate buffer), a soothing agent (e.g. benzalkonium chloride and procaine hydrochloride), a stabilizer (e.g. human serum albumin and polyethylene glycol), a preservative (e.g. benzyl alcohol and phenol), an antioxidant, or the like. Usually, the resulting injectable preparation is filled in an appropriate ampoule.

The resulting preparation is safe and less toxic and therefore can be administered to human or mammals (such as rat, mouse, rabbit, sheep, pig, bovine, cat, dog, and monkey).

The dose of the antisense nucleotide may vary depending on a particular subject, subject organ, symptom, way of administration, and the like. However, in general, for example, the antisense nucleotide may be orally administered to a patient with osteoporosis patient (60 kg in weight) in a dose of about 0.1 mg to 100 mg per day, preferably about 1.0 to 50 mg per day, more preferably about 1.0 to 20 mg per day. In case of parenteral administration, the antisense nucleotide may be administered, for example, in the form of an injectable preparation, to a patient with osteoporosis (60 kg in weight), usually, in a dose of about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [15] Use of Anti-NQO2 Antibody

### (a) Diagnostic

The antibody against NQO2, etc. is capable of specifically recognizing NQO2, etc. and therefore can be used for quantitative determination of NQO2, etc. in a solution to be tested, in particular, for quantitative determination by sandwich immunoassay. That is, the present invention provides, for example:
(i) a method of quantitatively determining NQO2, etc. in a solution to be tested, which comprises competitively reacting the antibody with the solution to be tested and a labeled NQO2, etc., and measuring the ratio of the labeled NQO2, etc. bound to the antibody; and
(ii) a method of quantitatively determining NQO2, etc. in a solution to be tested, which comprises reacting the solution to be tested with the antibody insolubilized on a carrier and the labeled antibody simultaneously or continuously, and measuring the activity of the labeling agent on the insolubilized carrier.

In the method (ii), the insolubilized antibody and the labeled antibody preferably have antigen recognition sites, respectively, in such a manner that they do not interfere with each other in binding to NQO2, etc. (for example, one antibody recognizes the N-terminal region of NQO2, etc., while the other antibody reacts with the C-terminal region of NQO2, etc.).

In addition to the determination of NQO2, etc. by using the monoclonal antibody to NQO2, etc. (hereinafter referred to as the monoclonal antibody of the present invention in some cases), the detection by tissue staining can also be employed. For these purposes, the antibody itself may be used as it is, or F(ab')₂, Fab' or a Fab fraction of the antibody molecule may be used. The measurement using the antibody against NQO2, etc. is not specifically limited and any method can be used in so far as the method comprises quantitatively detecting an antibody, an antigen or an antibody-antigen complex corresponding to the amount of an antigen (e.g. the amount of NQO2) in a solution to be tested by a chemical or physical means, and calculating the quantity by using a standard curve prepared with standard solutions each containing a known amount of the antigen. For example, nephrometry, competitive method, immunometric method or sandwich method is preferably used, and in view of sensitivity and specificity, the sandwich method as described hereinafter is particularly preferred.

Examples of the labeling agent for use in the measurement using a labeled substance include radioisotopes, enzymes, fluorescent substances, and luminescent substances. Examples of the radioisotope include [¹²⁵I], [¹³¹I], [³H], and [¹⁴C]. The enzyme is preferably stable with high specific activity and examples thereof include β-galactosidase, β-glucosidase, alkaline phosphatase, peroxidase, and malate dehydrogenase. Examples of the fluorescent substance include fluorescamine and fluorescein isothiocyanate. Examples of the luminescent substance include luminol, luminol derivatives, luciferin, and lucigenin. A biotin-avidin system may also be used for the binding of the antibody or the antigen to the labeling agent.

The insolubilization of an antigen or an antibody may be performed by using physical adsorption or by a chemical bonding method that is usually employed for insolubilization or immobilization of proteins or enzymes. Examples of the carrier include insoluble polysaccharides such as agarose, dextran and cellulose; synthetic resins such as polystyrene, polyacrylamide and silicone; and glass.

In the sandwich method, the amount of NQO2 in a solution to be tested can be determined by reacting the solution to be tested with an insolubilized monoclonal antibody (1st reaction) and further reacting the solution to be tested with a labeled monoclonal antibody (2nd reaction); and then measuring the activity of the labeling agent on the insoluble carrier. The 1st reaction and the 2nd reaction may be performed in the reverse order or simultaneously, or they may be performed at certain time intervals. The type of the labeling agent and the insolubilization method may be the same as those described above.

Further, in the immunoassay using the sandwich method, the antibody to be immobilized or labeled is not always limited to a single type, and a mixture of two or more types of antibodies can be used for the purpose of improving the measurement sensitivity.

In the measurement of NQO2, etc. by the sandwich method of the present invention, monoclonal antibodies whose binding sites to NQO2, etc. differ from each other are preferably used in the 1st and 2nd reactions, respectively. That is, if the antibody for use in the 2nd reaction recognizes the C-terminal region of NQO2, the antibody for use in the 1st reaction preferably recognizes any other region, for example, the N-terminal region.

The monoclonal antibody may also be used for a measurement system other than the sandwich system, for example, competitive method, immunometric method and nephrometry. In the competitive method, the amount of an antigen in a solution to be tested can be determined by competitively reacting the antigen in the solution to be tested and the labeled antigen with an antibody, then separating the unreacted labeled antigen (F) from the labeled antigen bound to the antibody (B) (B/F separation), and measuring the labeled amount of either B or F. The process to be used in this reaction includes a liquid phase system, wherein a soluble antibody is used as the antibody and polyethylene glycol, a second antibody against the above antibody, etc. is used for the B/F separation, as well as a solid phase system, wherein an immobilized antibody is used as a 1st antibody, or a soluble antibody used as a 1st antibody and an immobilized antibody is used as a 2nd antibody.

In the immunometric method, the amount of an antigen in an solution to be tested is determined by competitively reacting the antigen in the solution to be tested and the immobilized antigen with a certain amount of a labeled antibody and then separating the solid phase from the liquid phase; or by reacting the antigen in the solution to be tested with an excess amount of the labeled antibody, adding the immobilized antigen to bind the unreacted labeled antibody to the solid phase and then separating the solid phase from the liquid phase, followed by measuring the labeled amount in either phase.

In the nephrometry, an antigen-antibody reaction is performed in a gel or solution, and then the amount of the resulting insoluble precipitate is measured. Even when the amount of an antigen in a solution to be tested is so small that a precipitate is obtained in only a small amount, laser nephrometry based on laser beam scattering is preferably used.

For the application of each of the above immunological measurement methods, there is no need to set special conditions, operations, or the like. The system for measuring NQO2 or a salt thereof according to the present invention can be constructed by taking account of normal conditions and operation techniques of the above each method as well as conventional technical consideration of a person skilled in the art. The details of such general techniques are available from reviews or books [e.g. see Hiroshi Irie (ed), "Radioimmunoassay" Kodansha, 1974; Hiroshi Irie (ed), "Radioimmunoassay, Second Series", Kodansha, 1979; Eiji Ishikawa et al. (ed), "Enzyme Immunoassay", Igaku Shoin, 1978; Eiji Ishikawa et al. (ed), "Enzyme Immunoassay", Second Edition, Igaku Shoin, 1982; Eiji Ishikawa et al. (ed), "Enzyme Immunoassay", Third Edition, Igaku Shoin, 1987; "Methods in Enzymology", Academic Press, Vol. 70, Immunochemical Techniques (Part A); ibid. Vol. 73, Immunochemical Techniques (Part B); ibid. Vol. 74, Immunochemical Techniques (Part C); ibid. Vol. 84, Immunochemical Techniques (Part D: Selected Immunoassays); ibid. Vol. 92, Immunochemical Techniques (Part E: Monoclonal Antibodies and General Immunoassay Methods); ibid. Vol. 121, Immunochemical Techniques (Part I: Hybridoma Technology and Monoclonal Antibodies); etc.].

By using the antibody to NQO2, etc. in the above manner, NQO2, etc. can be quantitatively determined with high sensitivity.

Further, NQO2-related bone diseases can also be diagnosed by determining the amount of NQO2, etc. in vivo with the antibody.

For example, when a concentration of NQO2, etc. is found to be higher or lower than the normal value by using the antibody, the subject can be diagnosed as suffering from or having a high risk of any of a variety of bone diseases, for example, non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; articular diseases typically including cartilage diseases such as osteoarthritis and rheumatoid arthritis; osteopetrosis; and osteochondroma.

Further, the antibody can also be used for specific detection of NQO2, etc. in samples to be tested such as body fluids and tissues. Furthermore, the antibody can also be used for the preparation of an antibody column for purifying NQO2, etc., the detection of NQO2, etc. in each fraction during purification, the analysis of the behavior of NQO2, etc. in cells to be tested, and the like.

### (b) Drugs for Preventing or Treating Bone Diseases

The antibody to NQO2, etc. can specifically recognize NQO2, etc. and bind thereto to inhibit the reductase activity of NQO2. Therefore, the antibody is useful as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent.

Further, for example, the antibody is useful as an agent for preventing or treating bone diseases. More specifically, the antibody is useful as a safe and less toxic agent for preventing or treating a variety of bone diseases, for example, non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; and articular diseases typically including cartilage diseases such as osteoarthritis and rheumatoid arthritis. The antibody can also be used as a bone tissue-repairing agent after a surgery of multiple myeloma, lung cancer, breast cancer, or the like. In the field of dentistry, the antibody can also be used for treatment of periodontosis, repair of a periodontosis-induced periodontium defect, stabilization of an artificial root of a tooth, residual ridge formation, repair of cleft palate, or the like.

When the antibody is used as the above prophylactic or therapeutic agent, the antibody may be used as it is in the form of a liquid agent, may be formed into a pharmaceutical composition in a suitable form, or may be used in combination with a supplemental agent for enhancing the uptake.

For example, the antibody may be used orally in the form of a tablet, if necessary, coated with sugar, a capsule, an elixir, microcapsules, or the like, or parenterally in the form of an injectable preparation such as an aseptic solution and suspension in water or another pharmaceutically acceptable liquid. For example, the antibody may be mixed with a physiologically acceptable known carrier, flavoring agent, excipient, vehicle, antiseptic, stabilizer, binder, or the like, in order to form a preparation in a generally acceptable unit dosage form. The content of the active ingredient in such a preparation may be set so as to provide an appropriate dose within a specified range.

Examples of the additive which may be mixed in a tablet, a capsule or the like include a binder such as gelatin, corn starch, tragacanth, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage form is a capsule, it may further contain a liquid carrier such as fats and oils in addition to the above materials. A sterile composition for injection can be formulated according to a conventional preparation procedure such as dissolution or suspension of the active substance, a naturally occurring vegetable oil such as sesame oil and coconut oil, and the like in a vehicle such as water for injection. Examples of the aqueous liquid for injection to be used include a physiological saline and an isotonic solution containing glucose or another auxiliary agent (such as D-sorbitol, D-mannitol and sodium chloride). Such an aqueous liquid can be used in combination with an appropriate dissolution aid such as an alcohol (such as ethanol), a polyalcohol (such as propylene glycol and polyethylene glycol), and a nonionic surfactant (such as polysorbate 80™ and HCO-50). Examples of the oily liquid to be used include sesame oil and soybean oil. The oily liquid can be used in combination with a dissolution aid such as benzyl benzoate and benzyl alcohol.

The above prophylactic or therapeutic agent may also be formulated with a buffer (such as a phosphate buffer and a sodium acetate buffer), a soothing agent (such as benzalkonium chloride and procaine hydrochloride), a stabilizer (such as human serum albumin and polyethylene glycol), a preservative (such as benzyl alcohol and phenol), an antioxidant, or the like. Usually, the resulting injectable preparation is filled in an appropriate ampoule.

The resulting preparation thus obtained is safe and less toxic and therefore can be administered to a human being or mammals (such as rat, mouse, rabbit, sheep, pig, bovine, cat, dog, and monkey).

The dose of the antibody may vary depending on a particular subject, subject organ, symptom, way of administration, and the like. For example, the antibody may be orally administered to a patient with osteoporosis (60 kg in weight) in a dose of generally about 0.1 to 100 mg per day, preferably about 1.0 to 50 mg per day, more preferably about 1.0 to 20 mg per day. The antibody may also be parenterally administered, for example, in the form of an injectable preparation, to a patient with osteoporosis (60 kg in weight) in a dose of generally about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

Further, the antibody to NQO2, etc., which specifically recognizes NQO2, etc., can also be used as a targeting drug in combination with the above NQO2 activity-inhibiting or promoting agent to deliver the NQO2 activity-inhibiting or promoting agent to NQO2.

### [16] Use of Nucleotide Encoding NQO2, etc.

### (a) Diagnostic

The nucleotide containing a NQO2, etc.-encoding nucleotide can be used as a probe. Such a probe can be used to detect an abnormality (a genetic abnormality) of DNA or mRNA encoding NQO2, etc. in a human being or other mammals (such as rat, mouse, rabbit, sheep, pig, bovine, cat, dog, and monkey). Therefore, the nucleotide is useful as a gene diagnosis agent for diagnosing damage or mutation of the DNA or the mRNA, a reduction in expression of the DNA or the mRNA, or an increase or an excessive expression of the DNA or the mRNA.

For example, the gene diagnosis using the polynucleotide can be performed by using known northern hybridization or PCR-SSCP method (Genomics, Vol. 5, pp 874-879, 1989; Proceedings of the National Academy of Sciences of the United States of America, Vol. 86, pp 2766-2770, 1989).

For example, if the amount of the DNA or the mRNA for NQO2, etc. is found to be higher or lower than a normal value by using the polynucleotide, the subject can be diagnosed as suffering from or having a high risk of a variety of bone diseases, for example, non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; articular diseases typically including cartilage diseases such as osteoarthritis and rheumatoid arthritis; osteopetrosis; and osteochondroma.

### (b) Agent for Preventing or Treating Bone Diseases

The polynucleotide having the nucleotide sequence that encodes NQO2 or its peptide fragment having NQO2 enzyme activity can be used for enhancing the capability of synthesizing NQO2 protein and enriching the amount of NQO2 protein in cells of a human being or other mammals by using the polynucleotide inserted into an expression vector having a promoter operable in cells of a human being or other mammals and introducing the vector into the human being or other mammals. Therefore, in order to control conditions in an animal showing an abnormal increase in osteogenesis, osteoblast differentiation, chondrogenesis, chondrocyte differentiation, or BMP action due to a reduction in NQO2 expression, NQO2 defect caused by the mutation of a NQO2 gene, or production of less active NQO2, the polynucleotide is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

The polynucleotide can also be used as an agent for preventing or treating bone diseases such as osteopetrosis and osteochondroma.

When the polynucleotide is used as the above prophylactic or therapeutic agent, the nucleotide may be inserted into a suitable vector such as a retrovirus vector, an adenovirus vector and an adenovirus-associated virus vector before use according to a conventional means. The polynucleotide may be administered as it is or with a supplemental agent for enhancing the uptake by using a gene gun or a catheter such as a hydrogel catheter.

For example, the polynucleotide may be used orally in the form of a tablet, if necessary, with sugar coating, a capsule, an elixir, microcapsules, or the like, or parenterally in the form of an injectable preparation such as an aseptic solution and suspension in water or another pharmaceutically acceptable liquid. For example, the polynucleotide may be mixed with a physiologically acceptable known carrier, flavoring agent, excipient, vehicle, antiseptic, stabilizer, binder, or the like, in order to form a preparation in a generally acceptable unit dosage form. The content of the active ingredient in such a preparation may be set so as to provide an appropriate dose within a specified range.

Examples of the additive which may be mixed in a tablet, a capsule or the like include a binder such as gelatin, corn starch, tragacanth, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage form is a capsule, it can further contain a liquid carrier such as fats and oils in addition to the above materials. A sterile composition for injection may be formulated according to a conventional preparation procedure such as dissolving or suspending the active substance, a naturally occurring vegetable oil such as sesame oil and coconut oil, and the like in a vehicle such as water for injection. Examples of the aqueous liquid for injection include a physiological saline and an isotonic solution containing glucose or another auxiliary agent (such as D-sorbitol, D-mannitol and sodium chloride). Such an aqueous liquid may be used in combination with an appropriate dissolution aid, for example an alcohol (such as ethanol), a polyalcohol (such as propylene glycol and polyethylene glycol), and a nonionic surfactant (such as polysorbate 80™ and HCO-50). Examples of the oily liquid include sesame oil and soybean oil. The oily liquid may be used in combination with a dissolution aid such as benzyl benzoate and benzyl alcohol.

Further, the above prophylactic or therapeutic agent may also be formulated with a buffer (such as a phosphate buffer and a sodium acetate buffer), a soothing agent (such as benzalkonium chloride and procaine hydrochloride), a stabilizer (such as human serum albumin and polyethylene glycol), a preservative (such as benzyl alcohol and phenol), an antioxidant, or the like. The resulting injectable preparation is usually filled in an appropriate ampoule.

The resulting preparation is safe and less toxic and therefore can be administered to a human being or other mammals (such as rat, mouse, rabbit, sheep, pig, bovine, cat, dog, and monkey).

The dose of the polynucleotide may vary depending on a particular subject, subject organ, symptom, way of administration, and the like. For example, the polynucleotide may be orally administered to a patient with osteopetrosis (60 kg in weight) in a dose of generally about 0.1 mg to 100 mg per day, preferably about 1.0 to 50 mg per day, more preferably about 1.0 to 20 mg per day. The polynucleotide may also be parenterally administered, for example, in the form of an injectable preparation, to a patient with osteopetrosis (60 kg in weight) in a dose of generally about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [17] Use of NQO2, etc.

NQO2, its peptide fragment having NQO2 enzyme activity or a salt thereof can be administered to an animal to enhance the NQO2 activity in the animal body, similarly to the above polynucleotide. Therefore, NQO2, etc. is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

Further, NQO2, etc. can also be used as an agent for preventing or treating bone diseases such as osteopetrosis and osteochondroma.

When NQO2, etc. are used as the above prophylactic or therapeutic agent, they may be used as they are in the form of a liquid agent, may be formed into a pharmaceutical composition in a suitable form, or may be administered in combination with a supplemental agent for enhancing the uptake.

For example, NQO2, etc. may be used orally in the form of a tablet, if necessary, with sugar coating, a capsule, an elixir, microcapsules, or the like, or parenterally in the form of an injectable preparation such as an aseptic solution and suspension in water or another pharmaceutically acceptable liquid. For example, the NQO2, etc. may be mixed with a physiologically acceptable known carrier, flavoring agent, excipient, vehicle, antiseptic, stabilizer, binder, or the like, in order to form a preparation in a generally acceptable unit dosage form. The content of the active ingredient in such a preparation may be set so as to provide an appropriate dose within a specified range.

Examples of the additive which may be mixed in a tablet, a capsule or the like include a binder such as gelatin, corn starch, tragacanth, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage form is a capsule, it can further contain a liquid carrier such as fats and oils in addition to any of the above materials. A sterile composition for injection may be formulated according to a conventional preparation procedure such as dissolving or suspending the active substance, a naturally occurring vegetable oil such as sesame oil and coconut oil, and the like in a vehicle such as water for injection. Examples of the aqueous liquid for injection include a physiological saline and an isotonic solution containing glucose or another auxiliary agent (such as D-sorbitol, D-mannitol and sodium chloride). Such an aqueous liquid may be used in combination with an appropriate dissolution aid such as an alcohol (such as ethanol), a polyalcohol (such as propylene glycol and polyethylene glycol), and a nonionic surfactant (such as polysorbate 80™ and HCO-50). Examples of the oily liquid include sesame oil and soybean oil. The oily liquid may be used in combination with a dissolution aid such as benzyl benzoate and benzyl alcohol.

Further, the above prophylactic or therapeutic agent may also be formulated with a buffer (such as a phosphate buffer and a sodium acetate buffer), a soothing agent (such as benzalkonium chloride and procaine hydrochloride), a stabilizer (such as human serum albumin and polyethylene glycol), a preservative (such as benzyl alcohol and phenol), an antioxidant, or the like. Usually, the resulting injectable preparation is filled in an appropriate ampoule.

The resulting preparation is safe and less toxic and therefore can be administered to a human being or other mammals (such as rat, mouse, rabbit, sheep, pig, bovine, cat, dog, and monkey).

The dose of NQO2, etc. may vary depending on a particular subject, subject organ, symptom, way of administration, and the like. For example, NQO2, etc. may be orally administered to an osteopetrosis patient (60 kg in weight) in a dose of generally about 0.1 mg to 100 mg per day, preferably about 1.0 to 50 mg per day, more preferably about 1.0 to 20 mg per day. NQO2, etc. may also be parenterally administered, for example, in the form of an injection, to the osteopetrosis patient (60 kg in weight) in a dose of generally about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [18] Method for Screening Compounds Capable of Changing Expression Amount of NQO2, etc.

The present invention also provides a method for screening a compound or a salt thereof that can change the expression amount of NQO2, etc. In this method, a screening for a compound or a salt thereof capable of changing the expression amount of NQO2, etc. is performed by using the nucleotide having a nucleotide sequence that encodes the NQO2, etc. as a probe or primer.

That is, for example, the present invention is directed to a method for screening a compound capable of changing the expression amount of NQO2, etc., which comprises determining the amount of mRNA of NQO2, etc. contained in (i) (a) blood, (b) a specific organ for expressing NQO2 or (c) a tissue or cells isolated from the organ of a human being or mammals, or in (ii) a transformant or the like into which the polynucleotide containing a nucleotide sequence encoding NQO2 or its peptide fragment having NQO2 activity is introduced by using the nucleotide containing a nucleotide sequence encoding NQO2, etc.

Specifically, the amount of mRNA of NQO2, etc. is determined as follows:
(i) A normal or disease model of a non-human mammal (e.g. mouse, rat, rabbit, sheep, pig, bovine, cat, dog, and monkey, more specifically osteoporosis mouse, osteopetrosis mouse, dementia rat, obesity mouse, arteriosclerosis rabbit, and cancer-bearing mouse) is given an agent (e.g. an oxidizing agent, an antioxidant, vitamins, a heavy metal, an anti-dementia agent, an antihypertensive agent, an antitumor agent, and an anti-obesity agent) or physical stress (e.g. ultraviolet radiation, submerging stress, electric shock, dark-light stress, and low temperature); After a certain time period, blood, a specific organ (e.g. brain, liver or kidney), or a tissue or cells isolated from an organ are obtained. Or, human blood or organ, or a tissue or cells isolated from the organ are obtained.
   The amount of mRNA of NQO2, etc. contained in the cells of the like thus obtained can be determined by extracting mRNA from the cells or the like, for example, according to a conventional method, and using quantitative determination method, for example, TaqMan PCR technique or the like. Or, it can be analyzed by northern blotting according to a per se known technique.
(ii) A transformant expressing NQO2, etc. is prepared by the method as described above; and mRNA for NQO2, etc. contained in the transformant can be quantitatively determined or analyzed according to the same manner.

The screening for a compound capable of changing the expression amount of NQO2, etc. can be performed by:
(i) administering a test compound to a normal or disease model of a non-human mammal at a certain time period (30 minutes to 24 hours, preferably 30 minutes to 12 hours, more preferably one hour to six hours) before giving the agent or the physical stress, or at a certain time period (30 minutes to three days, preferably one hour to two days, more preferably one hour to 24 hours) after giving the agent or the physical stress, or at the same time of giving the agent or the physical stress, and determining or analyzing the amount of mRNA of NQO2, etc. contained in the cells at a certain time period (30 minutes to three days, preferably one hour to two days, more preferably one hour to 24 hours) after administering the test compound; or
(ii) mixing a test compound in a culture medium when culturing a transformant or a human-derived cells according to a conventional method, and determining or analyzing the amount of the mRNA of NQO2, etc. in the transformant or the like after culturing for a certain time period (one day to seven days, preferably one day to three days, more preferably two days to three days).

The compound or a salt thereof obtained by using the screening method has an action of changing the expression amount of NQO2, etc. Specifically, such a compound or a salt thereof is (a) a compound that increases the expression amount of NQO2, etc. to promote the NQO2 activity or (b) a compound that decreases the expression amount of NQO2, etc. to inhibit the NQO2 activity.

Examples of such a compound include peptides, proteins, non-peptidic compounds, synthetic compounds, and fermentation products. They may be novel compounds or known compounds.

The compound or a salt thereof that increases the expression amount of NQO2, etc. is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

Further, the compound or a salt is also useful as an agent for preventing or treating bone diseases such as osteopetrosis and osteochondroma.

The compound or a salt that decreases the expression amount of NQO2, etc. is useful as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent.

Further, the compound or a salt is also useful as a safe and less toxic agent for preventing or treating bone or articular diseases, more specifically, for preventing or treating a variety of bone diseases, for example, non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; and articular diseases typically including cartilage diseases such as osteoarthritis and rheumatoid arthritis. The compound or a salt thereof can also be used as a bone tissue-repairing agent after a surgery of multiple myeloma, lung cancer, breast cancer, or the like. In the field of dentistry, the compound or a salt thereof may also be used for treatment of periodontosis, repair of a periodontosis-induced periodontium defect, stabilization of an artificial root of a tooth, residual ridge formation, repair of cleft palate, or the like.

When the compound or a salt thereof capable of changing the expression amount of NQO2 obtained by using the above screening method is used as a pharmaceutical composition, it is performed according to a conventional method. For example, the compound or a salt may be formulated into a tablet, a capsule, an elixir, microcapsules, an aseptic solution or suspension similarly to the preparation containing a compound or a salt thereof that promotes or inhibits NQO2 reductase activity.

The resulting preparation is safe and less toxic and therefore can be administered to a human being or mammals (e.g. rat, mouse, rabbit, sheep, pig, bovine, cat, dog, and monkey).

The dose of the compound or a salt thereof may vary depending on a particular subject, subject organ, symptom, way of administration, and the like. For example, the compound that decreases the expression amount of NQO2, etc. may be orally administered to a patient with osteoporosis (60 kg in weight) in a dose of generally about 0.1 mg to 100 mg per day, preferably about 1.0 to 50 mg per day, more preferably about 1.0 to 20 mg per day. It is favorable that the compound that decreases the expression amount of NQO2, etc. may be parenterally administered, for example, in the form of an injectable preparation, to a patient with osteoporosis (60 kg in weight) in a dose of generally about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [19] Method for Screening Compounds Capable of Changing Activity of NQO2 Promoter

The present invention also provides a method for screening a compound or a salt thereof that can regulate an activity of an NQO2 promoter, which comprises measuring the activity of the promoter in both cases where a test compound is administered and no test compound is administered in a reporter gene assay using the NQO2 promoter. NQO2 is an enzyme involved in oxidative stress response or xenobiotic metabolism. In the vicinity of a promoter of a NQO2 gene, two types of cis-elements, i.e., an antioxidant response element (ARE) and a xenobiotic response element (XRE) are arranged. The NQO2 promoter used herein may include a promoter containing these cis-elements.

The reporter gene assay can be performed by using the known methods disclosed in The Journal of Biological Chemistry (J. B. C.), Vol. 272, 22800-22808, 1997 and Development, Vol. 124, 793-804, 1997.

Examples of the test compound include peptides, proteins, biological non-peptidic compounds (such as saccharides and lipids), synthetic compounds, microorganism culture products, cell extracts, plant extracts, and animal tissue extracts. They may be novel compounds or known compounds.

The promoter activity may be measured by examining the quantity of the expression amount of the reporter gene. For example, such a quantity is determined by northern blotting, Reverse transcription-polymerase chain reaction (RT-PCR), TaqMan polymerase chain reaction or an analogues method.

In this method, a test compound that increases the expression amount of the reporter gene by about 20% or more, preferably 30% or more, more preferably about 50% or more may be selected as a NQO2 promoter activity-enhancing compound, as a result of comparing the case with administration of the test compound with the case without administration of the test compound.

On the other hand, a test compound that decreases the expression amount of the reporter gene by about 20% or more, preferably 30% or more, more preferably about 50% or more may be selected as a NQO2 promoter activity-inhibiting compound, as a result of comparing the case with administration of the test compound with the case without administration of the test compound.

The compound or a salt thereof that increases the NQO2 promoter activity is useful as an osteogenesis-inhibiting agent, an osteoblast (including the precursor osteoblast) differentiation-inhibiting agent, an osteoblast (including the precursor osteoblast) differentiation induction-inhibiting agent, a chondrogenesis-inhibiting agent, a chondrocyte (including the precursor chondrocyte) differentiation-inhibiting agent, a chondrocyte (including the precursor chondrocyte) differentiation induction-inhibiting agent, or a BMP action-inhibiting agent.

The compound or a salt is also useful as an agent for preventing or treating bone diseases such as osteopetrosis and osteochondroma.

The compound or a salt that decreases the NQO2 promoter activity is useful as an osteogenesis-promoting agent, an osteoblast (including the precursor osteoblast) differentiation-inducing agent, an osteoblast (including the precursor osteoblast) differentiation induction-promoting agent, a chondrogenesis-promoting agent, a chondrocyte (including the precursor chondrocyte) differentiation-inducing agent, a chondrocyte (including the precursor chondrocyte) differentiation induction-promoting agent, or a BMP action-enhancing agent.

The compound or a salt is also useful as a safe and less toxic agent for preventing or treating bone diseases, more specifically, for preventing or treating a variety of bone diseases such as non-metabolic bone diseases in the field of orthopedics, such as bone fractures, bone refracture, bone/spinal deformation, osteosarcoma, myeloma, bone dysplasia and scoliosis; metabolic bone diseases such as bone defect, osteoporosis, osteomalacia, rickets, fibrous osteitis, renal bone dystrophy, Paget's disease of bone, and rigid myelitis; and articular diseases typically including cartilage diseases such as osteoarthritis and rheumatoid arthritis. The compound or a salt thereof can also be used as a bone tissue-repairing agent after a surgery of multiple myeloma, lung cancer, breast cancer, or the like. In the field of dentistry, the compound or the salt thereof may also be used for treatment of periodontosis, repair of a periodontosis-induced periodontium defect, stabilization of an artificial root of a tooth, residual ridge formation, repair of cleft palate, or the like.

When the compound or a salt thereof capable of changing the activity of the NQO2 promoter obtained by using the above screening method is used as a pharmaceutical composition, it is performed according to a conventional method. For example, the compound or a salt may be formulated into a tablet, a capsule, an elixir, microcapsules, an aseptic solution or suspension similarly to the preparation containing a compound or a salt thereof that promotes or inhibits NQO2 reductase activity.

The resulting preparation is safe and less toxic and therefore can be administered to a human being or mammals (e.g. rat, mouse, rabbit, sheep, pig, bovine, cat, dog, and monkey).

The dose of the compound or a salt thereof may vary depending on a particular subject, subject organ, symptom, way of administration, and the like. For example, the compound that inhibits the activity of the NQO2 promoter may be orally administered to a patient with osteoporosis (60 kg in weight) in a dose of generally about 0.1 mg to 100 mg per day, preferably about 1.0 to 50 mg per day, more preferably about 1.0 to 20 mg per day. It is favorable that the compound that inhibit the activity of the NQO2 promoter may be parenterally administered, for example, in the form of an injectable preparation, to a patient with osteoporosis (60 kg in weight) in a dose of generally about 0.01 to about 30 mg per day, preferably about 0.1 to about 20 mg per day, more preferably about 0.1 to about 10 mg per day, although the dose may vary depending on a particular subject, subject organ, symptom, way of administration, and the like. Other animals may also take such a dose by converting their body weights into 60 kg.

### [20] Prevention or Treatment of Bone Diseases by Regulating Expression or Activity of NQO2

NQO2 has an osteogenesis-inhibiting action, an osteoblast (including a precursor osteoblast) differentiation-inhibiting action, an osteoblast (including a precursor osteoblast) differentiation induction-inhibiting action, a chondrogenesis-inhibiting action, a chondrocyte (including a precursor chondrocyte) differentiation-inhibiting action, a chondrocyte (including a precursor chondrocyte) differentiation induction-inhibiting action, or a BMP action-inhibiting action. Therefore, bone diseases can be prevented or treated by promoting or inhibiting the expression or activity of NQO2 in a body of a human being or other mammals suffering from or having a high risk of the bone diseases. Thus, the present invention also provides a method for preventing or treating a mammalian bone disease, which comprises inhibiting or promoting the expression or activity of NQO2 in the mammalian body to an effective extent for the prevention or treatment of the bone disease.

Examples of the means for inhibiting the expression or activity of NQO2 include, but are not limited to, administration of the compound obtained by the above screening method [18] that decreases the expression amount of NQO2 or the compound obtained by the above screening method [19] that inhibits the NQO2 promoter activity to an animal; or administration of the antisense nucleotide of NQO2 according to the above [14]; and further, administration of the compound that negatively regulates ARE or XRE in the promoter region of the NQO2 gene; or load of physical stress.

Likewise, examples of the means for inhibiting the activity of NQO2 include, but is not limited to, administration of the NQO2 activity inhibitor obtained by using the above screening method and/or kit [12] or by any other method to an animal according to the description of the above [13]; or administration of an antibody to NQO2, etc. according to the description of the above [15]; and further, administration of an NQO2 variant or a polynucleotide encoding the NQO2 variant that is prepared by a known method such as mutagen treatment or site-directed mutagenesis of NQO2 expression cells and maintains the affinity for a quinone substrate, while lacking the reductase activity, to an animal according to the methods described in the above [16] and [17] to an animal; or load of physical stress to inactivate NQO2.

The inhibition of the expression or activity of NQO2 can promote or enhance osteogenesis, differentiation or differentiation induction of the osteoblast (including the precursor osteoblast), chondrogenesis, differentiation or differentiation induction of the chondrocyte (including the precursor chondrocyte) or BMP action. Therefore, the above treatment is useful for preventing or treating a variety of bone diseases that are expected to be prevented or treated effectively by the above action. Examples of such bone diseases include bone fractures, bone refracture, bone/spinal deformation, osteosarcoma, myeloma, bone dysplasia, scoliosis, bone defect, osteoporosis, osteomalacia, rickets, fibrous osteitis, renal bone dystrophy, Paget's disease of bone, rigid myelitis, osteoarthritis, and rheumatoid arthritis.

Examples of the means for promoting the expression or activity of NQO2 include, but are not limited to, administration of the compound that is obtained by the above screening method [18] and increases the expression amount of NQO2 or the compound that is obtained by the above screening method [19] and enhances the NQO2 promoter activity to an animal; or administration of an polynucleotide encoding NQO2, etc. according to the description in the above [16]; and further, administration of the compound that positively regulates ARE or XRE in the promoter region of a NQO2 gene (e.g. an oxidizing agent, an antioxidant, vitamins, heavy metals, and another xenobiotic); or load of physical stress (e.g. ultraviolet radiation and oxidative stress).

Likewise, examples of the means for enhancing the activity of NQO2 include, but are not limited to, administration of the NQO2 activity-enhancing agent obtained by using the above screening method or kit as described in the section [12] or obtained by any other means to an animal according to the description of the above [13]; or administration of NQO2, its peptide fragment having NQO2 activity or a salt thereof according to the description of the above [17]; and further, administration of an NQO2 variant that is prepared by a known method such as mutagen treatment or site-directed mutagenesis of NQO2 expression cells and has a reductase activity higher than that of the wild type or a polynucleotide encoding the NQO2 variant to an animal according to the methods described in the above [16] and [17].

The promotion of the expression or activity of NQO2 can inhibit osteogenesis, differentiation or differentiation induction of the osteoblast (including the precursor osteoblast), chondrogenesis, differentiation or differentiation induction of the chondrocyte (including the precursor chondrocyte) or BMP action. Therefore, the above treatment is useful for preventing or treating a variety of bone diseases that are expected to be prevented or treated effectively by the inhibition of the above action. Examples of such bone diseases include osteopetrosis and osteochondroma.

The abbreviations for the bases or the amino acids used herein and the drawings are based on the recommendation by the IUPAC-IUB Commission on Biochemical Nomenclature or based on the conventional abbreviation in the field. Examples thereof are illustrated below. Unless otherwise specified, the amino acids are in the L isomers, if optical isomers exist.
DIVA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetracetic acid
SDS: Sodium dodecyl sulfate
EIA: Enzyme immunoassay
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine Gln: Glutamine
pGlu: Pyroglutamic acid
Me: Methyl
Et: Ethyl
Bu: Butyl
Ph: Phenyl
TC: Thiazolidine-4(R)-carboxamide

Each SEQ ID NO set forth in the SEQUENCE LISTING represents as follows:
SEQ ID NO: 1 represents a nucleotide sequence of a polynucleotide encoding human-derived NQO2;
SEQ ID NO: 2 represents an amino acid sequence of human-derived NQO2;
SEQ ID NO: 3 represents a nucleotide sequence of a polynucleotide encoding mouse-derived NQO2;
SEQ ID NO: 4 represents an amino acid sequence of mouse-derived NQO2;
SEQ ID NO: 5 represents a nucleotide sequence of a primer used in PCR in Example 1;
SEQ ID NO: 6 represents a nucleotide sequence of a primer used in PCR in Example 1;
SEQ ID NO: 7 represents a sequence of an antisense S-oligo DNA used in Example 2; and
SEQ ID NO: 8 represents a sequence of a sense S-oligo DNA used in Example 2.

The present invention will be more specifically illustrated by the following Examples, but such examples are not intended to limit the scope of the present invention. The genetic engineering technique using *Escherichia coli* is according to the method disclosed in Molecular Cloning.

### Example 1: Effect of Compound on NQO2 Activity

### (1) Construction of GST-NQO2 Fusion Protein Expression System

Entire RNA was prepared from a mouse osteoblastic cell line, MC3T3-E1, and cDNA was synthesized by using the oligo-dT primer. PCR was performed by using the cDNA as a template and two synthetic DNAs having the nucleotide sequences of SEQ ID NO: 5 and SEQ ID NO: 6, respectively, as primers to add the sequences recognized by restriction enzymes. The resulting DNA fragment was cleaved with restriction enzymes BamHI and SalI and inserted into the BamHI-SalI cleavage site of plasmid pGEX-4T-2 (Amersham Pharmacia Biotech). The nucleotide sequence was confirmed, and then *E. coli* DH5α (GIBCO BRL) carrying the plasmid was cultured in an LB medium. According to the protocol recommended by Amersham Pharmacia Biotech, expression was induced by IPTG, and then purification was performed by using Glutathione-Sepharose (Amersham Pharmacia Biotech). From 2 L of the LB medium, 3.0 mg of the fusion protein was obtained.

### (2) Effect of Compound on NQO2 Activity

Compound A having an osteogenesis-promoting action was examined for effects on the NQO2 activity by using the fusion protein obtained in the above (1). In the test using osteoblasts, the compound has an alkaline phosphatase-inducing action at a concentration of 1 µM. The reaction was performed at 25°C by adding 10 nM of the fusion protein to an aqueous solution containing 50 mM of Tris·HCl (pH 7.4), 200 mM of NaCl, 50 µM of 1-carbamoylmethyl-3-carbamoyl-1,4-dihydropyridine [a structural analog compound of NRH (dihydronicotinamide riboside) synthesized according to R. J. Knox et al. (Cancer Research, Vol. 60, pp.4179-4186, 2000)], and 30 µM of methyl red (Wako Pure Chemicals Industries, Ltd.). The decrease in absorbance at 436 nm was monitored to detect the reduction of methyl red. Compound A produced a 38% inhibitory action at 1 µM.

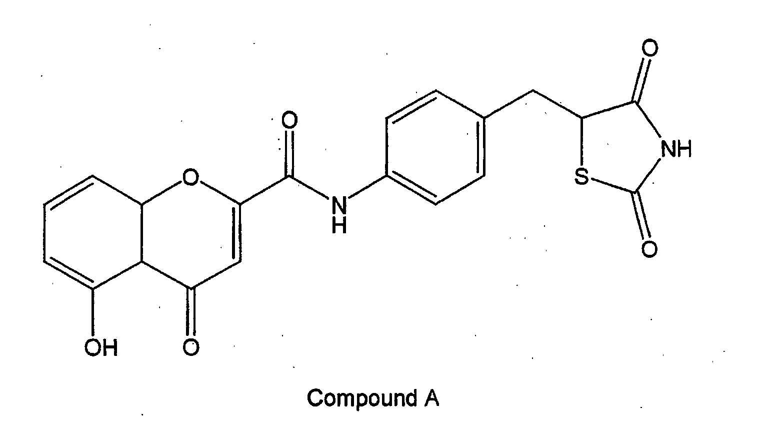

### Example 2: Osteoblast Differentiation Promotion Induced by Antisense DNA Introduction-Produced Inhibition of NQO2 Expression

The mouse osteoblastic cell line, MC3T3-E1, in a MEMα medium containing 10% fetal calf serum and 100 µg/ml of kanamycin was inoculated on a 48-well plate at 1.4 x 10⁴ cells per well and cultured overnight. To 100 µl of a serum-free αMEM were added 3 µl of FuGene 6 (Roche), 1.5 µg of the S-oligo DNA having the sequence of SEQ ID NO: 7 (antisense sequence to mouse NQO2 mRNA) and 1.5 µg of the S-oligo DNA having the sequence of SEQ ID NO: 8 (sense sequence to the mRNA) and the mixture was allowed to stand at room temperature for 15 minutes. To each well was added 22 µl of the mixture and incubated under 5% CO₂ at 37°C overnight. Thereafter, ascorbic acid was added at a final concentration of 50 µg/ml, and 10 mM of β-glycerophosphate was added. Incubation was performed for four days, and the activity of the alkaline phosphatase was measured according to Notoya et al. (Journal of Bone and Mineral Research 9: 395-400, 1994). The concentration of the protein in each cell homogenate was measured, and the activity values were corrected and shown in Fig. 1 by taking the activity obtained by the same procedure without addition of the DNA as 100%. In the cells where the sense sequence DNA was introduced, no difference was observed between such cells and cells without introduction of DNA. However, in the cells where the antisense DNA was introduced, their activity value was significantly higher than that of the cells without introduction of DNA (p<0.05). The result shows that the inhibition of the NQO2 expression promotes the differentiation of the osteoblast.

### Industrial Applicability

Osteogenesis-promoting or inhibiting drugs can be selected efficiently by using the screening method of the present invention.

The present application is based on Japanese Application No. 2001-128078, whose contents are entirely incorporated herein by reference.

The disclosures of all the above-mentioned literatures including patents and patent applications are incorporated herein by reference.

## Claims

1. A method for screening a compound acting on a bone, or a salt thereof, which comprises using NRH: quinone oxidoreductase (NQO2), a peptide fragment thereof, or a salt thereof.

2. The screening method according to claim 1, wherein the action on a bone is osteogenesis-promoting action, osteoblast differentiation-inducing action, osteoblast differentiation induction-promoting action, chondrogenesis-promoting action, chondrocyte differentiation-inducing action, chondrocyte differentiation induction-promoting action, or BMP action-enhancing action.

3. The screening method according to claim 1, wherein the action on a bone is osteogenesis-inhibiting action, osteoblast differentiation-inhibiting action, osteoblast differentiation induction-inhibiting action, chondrogenesis-inhibiting action, chondrocyte differentiation-inhibiting action, chondrocyte differentiation induction-inhibiting action, or BMP action-suppressing action.

4. The screening method according to claim 1, wherein a comparison is made between (i) the action on a bone in case where NQO2, a peptide fragment thereof, or a salt thereof is brought into contact with a substrate and (ii) that in case where NQO2, a peptide fragment thereof, or a salt thereof is brought into contact with the substrate and a test compound.

5. A kit for screening a compound or a salt thereof acting on a bone, which comprises NRH: quinone oxidoreductase (NQO2), a peptide fragment thereof, or a salt thereof.

6. A compound acting on a bone or a salt thereof obtainable by using the screening method according to claim 1 or the screening kit according to claim 5.

7. A bone-acting agent, which comprises a compound or a salt thereof obtainable by using the screening method according to claim 1 or the screening kit according to claim 5.

8. An agent for preventing and/or treating a bone disease, which comprises a compound having inhibitory action on the expression or activity of NRH: quinone oxidoreductase (NQO2), or a salt thereof.

9. The agent according to claim 8, which is an osteogenesis-promoting agent, an osteoblast differentiation-inducing agent, an osteoblast differentiation induction-promoting agent, a chondrogenesis-promoting agent, a chondrocyte differentiation-inducing agent, a chondrocyte differentiation induction-promoting agent, or a BMP action-enhancing agent.

10. The agent according to claim 8, which is an agent for preventing and/or treating bone fracture, bone refracture, bone/spinal deformation, osteosarcoma, myeloma, bone dysplasia, scoliosis, bone defect, osteoporosis, osteomalacia, rickets, fibrous osteitis, renal bone dystrophy, Paget's disease of bone, rigid myelitis, osteoarthritis, or rheumatoid arthritis.

11. An agent for preventing and/or treating a bone disease, which comprises a compound having promoting action on the expression or activity of NRH: quinone oxidoreductase (NQO2), or a salt thereof.

12. An agent for preventing and/or treating a bone disease, which comprises a polynucleotide having a nucleotide sequence complementary to a polynucleotide encoding NRH: quinone oxidoreductase (NQO2), or a fragment thereof.

13. An agent for preventing and/or treating a bone disease, which comprises an antibody against NRH: quinone oxidoreductase (NQO2), a peptide fragment thereof, or a salt thereof.

14. A diagnostic agent for a bone disease, which comprises an antibody against NRH: quinone oxidoreductase (NQO2), a peptide fragment thereof, or a salt thereof.

15. A method for preventing and/or treating a mammalian bone disease, which comprises inhibiting or promoting the expression or activity of NRH: quinone oxidoreductase (NQO2) in a mammalian body to an extent effective for preventing or treating the bone disease.

16. A method for preventing and/or treating a mammalian bone disease, which comprises administering a compound that inhibits or promotes the expression or activity of NRH: quinone oxidoreductase (NQO2), or a salt thereof, in an amount effective for preventing and/or treating the bone disease to a mammal.

17. Use of a compound that inhibits or promotes the expression or activity of NRH: quinone oxidoreductase (NQO2), or a salt thereof for the manufacture of an agent for preventing and/or treating a bone disease.
